# EUROPEAN PATENT SPECIFICATION

(11) **EP 1 737 879 B1**
(45) Date of publication and mention of the grant of the patent: **10.10.2012**
(21) Application number: 05778224.5
(22) Date of filing: 19.04.2005
(51) Int. Cl.: C12N 15/11, C07H 21/04

(54) **APTAMER-MEDIATED INTRACELLULAR DELIVERY OF THERAPEUTIC OLIGONUCLEOTIDES**
APTAMER-VERMITTELTE INTRAZELLULÄRE VERABREICHUNG VON THERAPEUTISCHEN OLIGONUKLEOTIDEN
DELIVRANCE INTRACELLULAIRE OLIGONUCLEOTIDES THERAPEUTIQUES MEDIEE PAR DES APTAMERES

(30) Priority: 19.04.2004 US 563428 P
(43) Date of publication of application: 03.01.2007
(73) Proprietor: Archemix LLC, San Francisco, CA 94111 (US)
(72) Inventor: EPSTEIN, David, Belmont, MA 02478 (US); PENDERGRAST, Shannon, Somerville, MA 02144 (US); KEEFE, Anthony, Dominic, Cambridge, MA 02141 (US)
(74) Representative: Clegg, Richard Ian
(86) International application number: PCT/US2005/013229
(87) International publication number: WO 2005/111238

(56) References cited:
- WO-A-02/16596
- HOEPRICH S ET AL: "Bacterial virus phi29 pRNA as a hammerhead ribozyme escort to destroy hepatitis B virus" GENE THERAPY, MACMILLAN PRESS LTD., BASINGSTOKE, GB, vol. 10, no. 15, 1 August 2003 (2003-08-01), pages 1258-1267, XP002328047 ISSN: 0969-7128
- LUPOLD S E ET AL: "Identification and characterization of nuclease-stabilized RNA molecules that bind human prostate cancer cells via the prostate-specific membrane antigen" CANCER RESEARCH, AMERICAN ASSOCIATION FOR CANCER RESEARCH, BALTIMORE, USA, DE, vol. 62, no. 14, 15 July 2002 (2002-07-15), pages 4029-4033, XP002308385
- DEVROE E ET AL: "Therapeutic potential of retroviral RNAi vectors" EXPERT OPINION ON BIOLOGICAL THERAPY, ASHLEY, LONDON, GB, vol. 4, 1 April 2004 (2004-04-01), pages 319-327, XP002989051 ISSN: 1471-2598
- TOULME J.-J.: 'Aptamers: Selected oligonucleotides for therapy' CURRENT OPINION IN MOLECULAR THERAPEUTICS vol. 2, no. 3, 2000, pages 318 - 324, XP001119703
- BOLOGNA ET AL: 'Linear polyethylenimine as a tool for comparative studies of antisense and short double-stranded RNA oligonucleotides' NUCLEOSIDES, NUCLEOTIDES & NUCLEIC ACIDS vol. 22, no. 5-8, 2003, pages 1729 - 1731, XP009029462
- TUSCHL ET AL: 'Small Interfering RNAs: a revolutionary tool for the analysis of gene function and gene therapy' MOLECULAR INTERVENTIONS vol. 2, no. 3, 2002, pages 158 - 167, XP002294002
- FLOEGE ET AL: 'Novel approach to specific growth factor inhibition in vivo' AMERICAN JOURNAL OF PATHOLOGY vol. 154, no. 1, 1999, pages 169 - 179, XP002947976
- OSTENDORF ET AL: 'VEGF165 mediates glomerular endothelial repair' JOURNAL OF CLINICAL INVESTIGATION vol. 104, no. 7, 1999, pages 913 - 923, XP002238360

## Description

### FIELD OF THE INVENTION

The invention relates generally to the field of nucleic acid therapeutics and more particularly to methods of modulating intracellular delivery of therapeutic oligonucleotides using an aptamer composition.

### BACKGROUND OF THE INVENTION

Aptamers are nucleic acid molecules having specific binding affinity to molecules through interactions other than classic Watson-Crick base pairing.

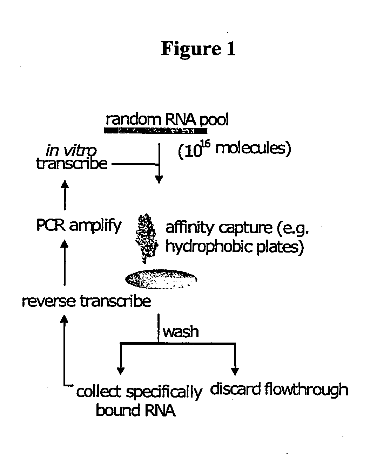

Aptamers, like peptides generated by phage display or monoclonal antibodies (mAbs), are capable of specifically binding to selected targets and modulating the target's activity, e.g., through binding, aptamers may block their target's ability to function. Created by an *in vitro* selection process from pools of random sequence oligonucleotides (Fig. 1), aptamers have been generated for over 100 proteins including growth factors, transcription factors, enzymes, immunoglobulins, and receptors. A typical aptamer is 10-15 kDa in size (30-45 nucleotides), binds its target with sub-nanomolar affinity, and discriminates against closely related targets (*e.g*., aptamers will typically not bind other proteins from the same gene family). A series of structural studies have shown that aptamers are capable of using the same types of binding interactions (*e.g*., hydrogen bonding, electrostatic complementarities, hydrophobic contacts, steric exclusion) that drive affinity and specificity in antibody-antigen complexes.

Aptamers have a number of desirable characteristics for use as therapeutics (and diagnostics) including high specificity and affinity, biological efficacy, and excellent pharmacokinetic properties. In addition, they offer specific competitive advantages over antibodies and other protein biologics, for example:

1) Speed and control. Aptamers are produced by an entirely *in vitro* process, allowing for the rapid generation of initial leads, including therapeutic leads. *In vitro* selection allows the specificity and affinity of the aptamer to be tightly controlled and allows the generation of leads, including leads against both toxic and non-immunogenic targets.

2) Toxicity and Immunogenicity. Aptamers as a class have demonstrated little or no toxicity or immunogenicity. In chronic dosing of rats or woodchucks with high levels of aptamer (10 mg/kg daily for 90 days), no toxicity is observed by any clinical, cellular, or biochemical measure. Whereas the efficacy of many monoclonal antibodies can be severely limited by immune response to antibodies themselves, it is extremely difficult to elicit antibodies to aptamers (most likely because aptamers cannot be presented by T-cells via the MHC, and the immune response is generally trained not to recognize nucleic acid fragments).

3) Administration. Whereas all currently approved antibody therapeutics are administered by intravenous infusion (typically over 2-4 hours), aptamers can also be administered by subcutaneous injection (aptamer bioavailability via subcutaneous administration is >80% in monkey studies (Tucker et al., J. Chromatography B. 732: 203-212, 1999)). This difference is primarily due to the comparatively low solubility and thus, large volumes necessary for most therapeutic MAbs. With good solubility (>150 mg/ml) and comparatively low molecular weight (aptamer: 10-50 kDa; antibody: 150 kDa), a weekly dose of aptamer may be delivered by injection in a volume of less than 0.5 ml. In addition, the small size of aptamers allows them to penetrate into areas of conformational constrictions that do not allow antibodies or antibody fragments to penetrate, presenting yet another advantage of aptamer-based therapeutics or prophylaxis.

4) Scalability and cost. Therapeutic aptamers are chemically synthesized and consequently can be readily scaled as needed to meet production demand. Whereas difficulties in scaling production are currently limiting the availability of some biologics and the capital cost of a large-scale protein production plant is enormous, a single large-scale oligonucleotide synthesizer can produce upwards of 100 kg per year and requires a relatively modest initial investment. The current cost of goods for aptamer synthesis at the kilogram scale is estimated at $500/g, comparable to that for highly optimized antibodies. Continuing improvements in process development are expected to lower the cost of goods to < $100 / g in five years.

5) Stability. Therapeutic aptamers are chemically robust. They are intrinsically adapted to regain activity following exposure to factors such as heat and denaturants and can be stored for extended periods (>1 yr) at room temperature as lyophilized powders. In contrast, antibodies must be stored refrigerated.

### Nucleic Acid Therapeutics

Nucleic acid therapeutics, such as oligonucleotides directed against intracellular targets (mRNA or protein), are powerful therapeutic agents. Examples of oligonucleotide therapeutic agents include: antisense oligonucleotides, which are short, single-stranded DNAs and RNAs that bind to complementary mRNA and inhibit translation or induce RNaseH-mediated degradation of the transcript; siRNA oligonucleotides, which are short, double-stranded RNAs that activate the RNA interference (RNAi) pathway leading to mRNA degradation; ribozymes, which are oligonucleotide-based endonucleases that are designed to cleave specific mRNA transcripts; and nucleic acid aptamers and decoys, which are non-naturally occurring oligonucleotides that bind to and block protein targets in a manner analogous to small molecule drugs.

Intracellular delivery of such therapeutic oligonucleotides is inefficient. Less than 2% of the administered dose of a therapeutic oligonucleotide is taken up into cells, either *in vitro* or *in vivo.* Uptake remains a significant barrier to the efficacy and therapeutic development of therapeutic oligonucleotide drugs (ODNs). *(See e.g.,* Lysik, M., and Wu-Pong, S. J. Pharmaceutical Sciences, 92: 1559 (2003). Existing delivery methods include both local and systemic administration of stabilized nucleic acids, liposomal-formulated oligonucleotides, and conjugated oligonucleotides in which the oligonucleotides are conjugated to peptides or small molecules that are subsequently transported into cells. The exact mechanisms for uptake of oligonucleotide drugs using these methods is unknown. While not intending to be bound by theory, it is thought the uptake of ODNs involves either specific receptor-mediated internalization of the nucleic acid/nucleic acid-conjugate or, in the case of liposomal-mediated transfection, diffusion of the positively charged liposome through the negatively charged membrane. The efficiency of these methods, when successful, remains low.

In addition to improving the efficiency of the uptake of ODNs, it would be beneficial to target oligonucleotides, including ODNs, to specific cell types. For example, oligonucleotide drugs could be targeted to cells activated in a diseased or pathological condition, but not healthy cells.

Intracellular uptake remains a significant problem in the development and use of therapeutic oligonucleotides directed toward destroying mRNA (siRNA, antisense, ribozymes) or blocking protein function (aptamers, decoys).

Accordingly, it would be beneficial to have materials and methods to modulate the intracellular delivery of therapeutic agents, such as therapeutic oligonucleotides.
Hoeprich et al (2003: Gene Therapy 10:1258-1267) used bacteriophage phi29 pRNA as a vector for imparting stability to ribozymes in vivo. They used a pRNA-based vector to carry hammerhead ribozymes that cleave the hepatitis B virus (HBV) polyA signal. The chimeric HBV-targeting ribozyme was connected to the pRNA 5'/3' ends as circularly permuted pRNA and two cis-cleaving ribozymes were used to flank and process the chimeric ribozyme. The hammerhead ribozyme was able to fold correctly while escorted by the pRNA. The chimeric ribozyme cleaved the polyA signal of HBV mRNA in vitro almost completely. Phi29 pRNA has a strong drive to form dimers, which are the building blocks of hexamers, leading Hoeprich et al to propose that formation of dimers or hexamers could allow multiple therapeutic agents to be carried.

### BRIEF DESCRIPTION OF THE DRAWINGS

Figure 1 is a schematic representation of the *in vitro* aptamer selection (SELEX™) process from pools of random sequence oligonucleotides.

Figure 2 is an illustration depicting various strategies for synthesis of high molecular weight PEG-nucleic acid conjugates.

Figures 3A-3I are illustrations depicting various design strategies for the bi-functional therapeutic oligonucleotides according to the invention.

Figures 4A-4C are illustrations depicting various aptamer sequences that are specific for CD4.

### SUMMARY OF THE INVENTION

The present invention provides materials and methods to mediate the intracellular delivery of therapeutic agents, such as therapeutic oligonucleotides. In one embodiment, the invention provides a bi-functional therapeutic oligonucleotide that includes a delivery aptamer, referred to herein as "Domain I", linked to a therapeutic oligonucleotide, referred to herein as "Domain II". The delivery aptamer and the therapeutic oligonucleotide are linked, for example, covalently or functionally through DNA/RNA or DNA/DNA duplex formation. In one embodiment of the invention, the bifunctional therapeutic oligonucleotide may be partly or wholly comprised of 2'-modified RNA or DNA such as 2'F, 2'OH, 2'OMe, 2'allyl, 2'MOE (methoxy-O-methyl) substituted nucleotides, and may contain PEG-spacers and abasic residues.

The invention provides therapeutic nucleic acid compositions that include a delivery aptamer sequence and a therapeutic oligonucleotide sequence linked by a linking moiety. The therapeutic oligonucleotide sequence is an siRNA oligonucleotide.

In one embodiment, the therapeutic oligonucleotide is an siRNA oligonucleotide covalently attached to the delivery aptamer through a 5'-3' phosphodiester linkage. In one embodiment, the coding strand of the siRNA oligonucleotide is covalently attached to the delivery aptamer through a 5'-3' phosphodiester linkage at the 5'-end of the delivery aptamer. In another embodiment, the coding strand of the siRNA oligonucleotide is covalently attached to the delivery aptamer through a 5'-3' phosphodiester linkage at the 3'-end of the delivery aptamer. In another embodiment, the coding and noncoding strands of the siRNA oligonucleotide are a contiguous sequence that is covalently attached to the delivery aptamer through a 5'-3' phosphodiester linkage to either the 5'- or 3' -end of the delivery aptamer.

In one embodiment, the siRNA oligonucleotide is specific for a target such as proliferating cell nuclear antigen (PCNA), BCL2, NFkB, VEGFR1, VEGFR2, VEGFR3, HER2/neu, c-Myc, REL-A, PTP1B, BACE, CHK1, PKC-alpha, EGFR, CHK-1, ERG2, Cyclin D1, CCND1 P, KCa, RAF1, MAPK1, MAPK8, MYB, KRAS2, KDR, IKKg, hTR, HRAS, FOS, GAB2, FLT1, EZH2 or CDK2.

In one embodiment, the therapeutically active oligonucleotide is an siRNA oligonucleotide that comprises or consists of one of the RNA sequences disclosed in Table 1 of PCT application WO 03/074654 (pages 140-173). Materials and methods for mediating intracellular delivery of an siRNA oligonucleotide having any one of the sequences disclosed in Table 1 of PCT application WO 03/074654 constitute specific embodiments of the present invention for which protection is hereby sought.

In one embodiment, the linking moiety is a nucleic acid moiety, a PNA moiety, a peptidic moiety, a disulfide bond or a polyethylene glycol moiety.

In one embodiment, the delivery aptamer sequence binds to a target such as a tumor antigen, a growth factor receptor, a cytokine receptor, a chemokine receptor, a G-protein coupled receptor, neurotensin (NTS-1) protein, a cytokine-cytokine receptor complex, or a viral coat protein. In one embodiment, the delivery aptamer sequence binds to a target such as PSMA, Her2/Neu, HERIII, CD4, cMet, EGFRI-III, MUC-1, CEA, CD6, CD19, CD22, CD23, CD33, CD44v6, CD56, VEGFRI, VEGFRII, NTS-1 protein, gp120 coat protein, gp41 fusion peptide, TENB2, HER2/neu (erb2), EGF receptor vIII, Cripto-1, or Alpha(v)beta(3/5).

In one embodiment, the therapeutic nucleic acid compositions also include a modified nucleotide, an abasic residue and/or a polyalkylene glycol residue.

In one embodiment, the therapeutic nucleic acid compositions include a nucleic acid sequence of SEQ ID NO:1-169 or SEQ ID NO: 170.

The invention also provides methods of modulating the intracellular delivery of a therapeutic agent in a subject by administering one or more of the therapeutic nucleic acid compositions of the invention to the subject.

In another aspect, the invention provides therapeutic nucleic acid compositions that include a delivery aptamer sequence, a therapeutic oligonucleotide, and a linking moiety, such that the linking moiety is not a nucleic acid molecule. In one embodiment, the linking moiety is a polyalkylene glycol. For example, the linking moiety is polyethylene glycol (PEG).

In one embodiment, the delivery aptamer sequence and the therapeutic oligonucleotide are linked by the PEG linking moiety of the therapeutic nucleic acid composition, such that the primary structure of the therapeutic nucleic acid composition is a linear arrangement in which the delivery aptamer is linked to a first terminus of the PEG linking moiety and the therapeutic oligonucleotide is linked to a second terminus of the PEG linking moiety.

### DETAILED DESCRIPTION OF THE INVENTION

The details of one or more embodiments of the invention are set forth in the accompanying description below. Although any methods and materials similar or equivalent to those described herein can be used in the practice or testing of the present invention, the preferred methods and materials are now described. Other features, objects, and advantages of the invention will be apparent from the description. In the specification, the singular forms also include the plural unless the context clearly dictates otherwise. Unless defined otherwise, all technical and scientific terms used herein have the same meaning as commonly understood by one of ordinary skill in the art to which this invention belongs. In the case of conflict, the present Specification will control.

### The SELEX™ Method

A suitable method for generating an aptamer is with the process entitled "Systematic Evolution of Ligands by EXponential Enrichment " ("SELEX™") generally depicted in Figure 1. The SELEX™ process is a method for the *in vitro* evolution of nucleic acid molecules with highly specific binding to target molecules and is described in, *e.g.,* U.S. Patent application Ser. No. 07/536,428, filed Jun. 11, 1990, now abandoned, U.S. Pat. No. 5,475,096 entitled "Nucleic Acid Ligands", and U.S. Pat. No. 5,270,163 (see also WO 91/19813) entitled "Nucleic Acid Ligands". Each SELEX-identified nucleic acid ligand is a specific ligand of a given target compound or molecule. The SELEX™ process is based on the unique insight that nucleic acids have sufficient capacity for forming a variety of two- and three-dimensional structures and sufficient chemical versatility available within their monomers to act as ligands (form specific binding pairs) with virtually any chemical compound, whether monomeric or polymeric. Molecules of any size or composition can serve as targets.

SELEX™ relies as a starting point upon a large library of single stranded oligonucleotides comprising randomized sequences derived from chemical synthesis on a standard DNA synthesizer. The oligonucleotides can be modified or unmodified DNA, RNA or DNA/RNA hybrids. In some examples, the pool comprises 100% random or partially random oligonucleotides. In other examples, the pool comprises random or partially random oligonucleotides containing at least one fixed sequence and/or conserved sequence incorporated within randomized sequence. In other examples, the pool comprises random or partially random oligonucleotides containing at least one fixed sequence and/or conserved sequence at its 5' and/or 3' end which may comprise a sequence shared by all the molecules of the oligonucleotide pool. Fixed sequences are sequences common to oligonucleotides in the pool which are incorporated for a pre-selected purpose such as, CpG motifs described further below, hybridization sites for PCR primers, promoter sequences for RNA polymerases (e.g., T3, T4, T7, and SP6), restriction sites, or homopolymeric sequences, such as poly A or poly T tracts, catalytic cores, sites for selective binding to affinity columns, and other sequences to facilitate cloning and/or sequencing of an oligonucleotide of interest. Conserved sequences are sequences, other than the previously described fixed sequences, shared by a number of aptamers that bind to the same target.

The oligonucleotides of the pool preferably include a randomized sequence portion as well as fixed sequences necessary for efficient amplification. Typically the oligonucleotides of the starting pool contain fixed 5' and 3' terminal sequences which flank an internal region of 30-50 random nucleotides. The randomized nucleotides can be produced in a number of ways including chemical synthesis and size selection from randomly cleaved cellular nucleic acids. Sequence variation in test nucleic acids can also be introduced or increased by mutagenesis before or during the selection/amplification iterations.

The random sequence portion of the oligonucleotide can be of any length and can comprise ribonucleotides and/or deoxyribonucleotides and can include modified or non-natural nucleotides or nucleotide analogs. See, e.g., U.S. Patent No. 5,958,691; U.S. Patent No. 5,660,985; U.S. Patent No. 5,958,691; U.S. Patent No. 5,698,687; U.S. Patent No. 5,817,635; U.S. Patent No. 5,672,695, and PCT Publication WO 92/07065. Random oligonucleotides can be synthesized from phosphodiester-linked nucleotides using solid phase oligonucleotide synthesis techniques well known in the art. See, e.g., Froehler et al., Nucl. Acid Res. 14:5399-5467 (1986) and Froehler et al., Tet. Lett. 27:5575-5578 (1986). Random oligonucleotides can also be synthesized using solution phase methods such as triester synthesis methods. See, e.g., Sood et al., Nucl. Acid Res. 4:2557 (1977) and Hirose et al., Tet. Lett., 28:2449 (1978). Typical syntheses carried out on automated DNA synthesis equipment yield 1014-1016 individual molecules, a number sufficient for most SELEX™ experiments. Sufficiently large regions of random sequence in the sequence design increases the likelihood that each synthesized molecule is likely to represent a unique sequence.

The starting library of oligonucleotides may be generated by automated chemical synthesis on a DNA synthesizer. To synthesize randomized sequences, mixtures of all four nucleotides are added at each nucleotide addition step during the synthesis process, allowing for random incorporation of nucleotides. As stated above, in one embodiment, random oligonucleotides comprise entirely random sequences; however, in other embodiments, random oligonucleotides can comprise stretches of nonrandom or partially random sequences. Partially random sequences can be created by adding the four nucleotides in different molar ratios at each addition step.

The starting library of oligonucleotides may be either RNA or DNA. In those instances where an RNA library is to be used as the starting library it is typically generated by transcribing a DNA *library in vitro* using T7 RNA polymerase or modified T7 RNA polymerases and purified. The RNA or DNA library is then mixed with the target under conditions favorable for binding and subjected to step-wise iterations of binding, partitioning and amplification, using the same general selection scheme, to achieve virtually any desired criterion of binding affinity and selectivity. More specifically, starting with a mixture containing the starting pool of nucleic acids, the SELEX™ method includes steps of: (a) contacting the mixture with the target under conditions favorable for binding; (b) partitioning unbound nucleic acids from those nucleic acids which have bound specifically to target molecules; (c) dissociating the nucleic acid-target complexes; (d) amplifying the nucleic acids dissociated from the nucleic acid-target complexes to yield a ligand-enriched mixture of nucleic acids; and (e) reiterating the steps of binding, partitioning, dissociating and amplifying through as many cycles as desired to yield highly specific, high affinity nucleic acid ligands to the target molecule. In those instances where RNA aptamers are being selected, the SELEX™ method further comprises the steps of: (i) reverse transcribing the nucleic acids dissociated from the nucleic acid-target complexes before amplification in step (d); and (ii) transcribing the amplified nucleic acids from step (d) before restarting the process.

Within a nucleic acid mixture containing a large number of possible sequences and structures, there is a wide range of binding affinities for a given target. A nucleic acid mixture comprising, for example, a 20 nucleotide randomized segment can have 4²⁰ candidate possibilities. Those which have the higher affinity constants for the target are most likely to bind to the target. After partitioning, dissociation and amplification, a second nucleic acid mixture is generated, enriched for the higher binding affinity candidates. Additional rounds of selection progressively favor the best ligands until the resulting nucleic acid mixture is predominantly composed of only one or a few sequences. These can then be cloned, sequenced and individually tested for binding affinity as pure ligands or aptamers.

Cycles of selection and amplification are repeated until a desired goal is achieved. In the most general case, selection/amplification is continued until no significant improvement in binding strength is achieved on repetition of the cycle. The method is typically used to sample approximately 1014 different nucleic acid species but may be used to sample as many as about 1018 different nucleic acid species. Generally, nucleic acid aptamer molecules are selected in a 5 to 20 cycle procedure. In one embodiment, heterogeneity is introduced only in the initial selection stages and does not occur throughout the replicating process.

In one embodiment of SELEX™, the selection process is so efficient at isolating those nucleic acid ligands that bind most strongly to the selected target, that only one cycle of selection and amplification is required. Such an efficient selection may occur, for example, in a chromatographic-type process wherein the ability of nucleic acids to associate with targets bound on a column operates in such a manner that the column is sufficiently able to allow separation and isolation of the highest affinity nucleic acid ligands.

In many cases, it is not necessarily desirable to perform the iterative steps of SELEX™ until a single nucleic acid ligand is identified. The target-specific nucleic acid ligand solution may include a family of nucleic acid structures or motifs that have a number of conserved sequences and a number of sequences which can be substituted or added without significantly affecting the affinity of the nucleic acid ligands to the target. By terminating the SELEX™ process prior to completion, it is possible to determine the sequence of a number of members of the nucleic acid ligand solution family.

A variety of nucleic acid primary, secondary and tertiary structures are known to exist. The structures or motifs that have been shown most commonly to be involved in non-Watson-Crick type interactions are referred to as hairpin loops, symmetric and asymmetric bulges, pseudoknots and myriad combinations of the same. Almost all known cases of such motifs suggest that they can be formed in a nucleic acid sequence of no more than 30 nucleotides. For this reason, it is often preferred that SELEX™ procedures with contiguous randomized segments be initiated with nucleic acid sequences containing a randomized segment of between about 20 to about 50 nucleotides and in some embodiments, about 30 to about 40 nucleotides. In one example, the 5'-fixed:random:3'-fixed sequence comprises a random sequence of about 30 to about 50 nucleotides.

The core SELEX™ method has been modified to achieve a number of specific objectives. For example, U.S. Patent No. 5,707,796 describes the use of SELEX™ in conjunction with gel electrophoresis to select nucleic acid molecules with specific structural characteristics, such as bent DNA. U.S. Patent No. 5,763,177 describes SELEX™ based methods for selecting nucleic acid ligands containing photo reactive groups capable of binding and/or photo-crosslinking to and/or photo-inactivating a target molecule. U.S. Patent No. 5,567,588 and U.S. Patent No. 5,861,254 describe SELEX™ based methods which achieve highly efficient partitioning between oligonucleotides having high and low affinity for a target molecule. U.S. Patent No. 5,496,938 describes methods for obtaining improved nucleic acid ligands after the SELEX™ process has been performed. U.S. Patent No. 5,705,337 describes methods for covalently linking a ligand to its target.

SELEX™ can also be used to obtain nucleic acid ligands that bind to more than one site on the target molecule, and to obtain nucleic acid ligands that include non-nucleic acid species that bind to specific sites on the target. SELEX™ provides means for isolating and identifying nucleic acid ligands which bind to any envisionable target, including large and small biomolecules such as nucleic acid-binding proteins and proteins not known to bind nucleic acids as part of their biological function as well as cofactors and other small molecules. For example, U.S. Patent No. 5,580,737 discloses nucleic acid sequences identified through SELEX™ which are capable of binding with high affinity to caffeine and the closely related analog, theophylline.

Counter- SELEX™ is a method for improving the specificity of nucleic acid ligands to a target molecule by eliminating, nucleic acid ligand sequences with cross-reactivity to one or more non-target molecules. Counter- SELEX™ is comprised of the steps of : (a) preparing a candidate mixture of nucleic acids; (b) contacting the candidate mixture with the target, wherein nucleic acids having an increased affinity to the target relative to the candidate mixture may be partitioned from the remainder of the candidate mixture; (c) partitioning the increased affinity nucleic acids from the remainder of the candidate mixture; (d) dissociating the increased affinity nucleic acids from the target; (e) contacting the increased affinity nucleic acids with one or more non-target molecules such that nucleic acid ligands with specific affinity for the non-target molecule(s) are removed; and (f) amplifying the nucleic acids with specific affinity only to the target molecule to yield a mixture of nucleic acids enriched for nucleic acid sequences with a relatively higher affinity and specificity for binding to the target molecule. As described above for SELEX™, cycles of selection and amplification are repeated as necessary until a desired goal is achieved.

One potential problem encountered in the use of nucleic acids as therapeutics and vaccines is that oligonucleotides in their phosphodiester form may be quickly degraded in body fluids by intracellular and extracellular enzymes such as endonucleases and exonucleases before the desired effect is manifest. The SELEX™ method thus encompasses the identification of high-affinity nucleic acid ligands containing modified nucleotides conferring improved characteristics on the ligand, such as improved *in vivo* stability or improved delivery characteristics. Examples of such modifications include chemical substitutions at the ribose and/or phosphate and/or base positions. SELEX™-identified nucleic acid ligands containing modified nucleotides are described, e.g., in U.S. Patent No. 5,660,985, which describes oligonucleotides containing nucleotide derivatives chemically modified at the 2' position of ribose, 5 position of pyrimidines, and 8 position of purines, U.S. Patent No. 5,756,703 which describes oligonucleotides containing various 2'-modified pyrimidines, and U.S. Patent No. 5,580,737 which describes highly specific nucleic acid ligands containing one or more nucleotides modified with 2'-amino (2'-NH2), 2'-fluoro (2'-F), and/or 2'-O-methyl (2'-OMe) substituents.

Modifications of the nucleic acid ligands contemplated in this invention include, but are not limited to, those which provide other chemical groups that incorporate additional charge, polarizability, hydrophobicity, hydrogen bonding, electrostatic interaction, and fluxionality to the nucleic acid ligand bases or to the nucleic acid ligand as a whole. Modifications to generate oligonucleotide populations which are resistant to nucleases can also include one or more substitute internucleotide linkages, altered sugars, altered bases, or combinations thereof. Such modifications include, but are not limited to, 2'-position sugar modifications, 5-position pyrimidine modifications, 8-position purine modifications, modifications at exocyclic amines, substitution of 4-thiouridine, substitution of 5-bromo or 5-iodo-uracil; backbone modifications, phosphorothioate or alkyl phosphate modifications, methylations, and unusual base-pairing combinations such as the isobases isocytidine and isoguanosine. Modifications can also include 3' and 5' modifications such as capping.

In one embodiment, oligonucleotides are provided in which the P(O)O group is replaced by P(O)S ("thioate"), P(S)S ("dithioate"), P(O)NR2 ("amidate"), P(O)R, P(O)OR', CO or CH2 ("formacetal") or 3'-amine (-NH-CH2-CH2-), wherein each R or R' is independently H or substituted or unsubstituted alkyl. Linkage groups can be attached to adjacent nucleotides through an -O-, -N-, or -S- linkage. Not all linkages in the oligonucleotide are required to be identical. As used herein, the term phosphorothioate encompasses one or more non-bridging oxygen atoms in a phosphodiester bond replaced by one or more sulfur atom.

In further embodiments, the oligonucleotides comprise modified sugar groups, for example, one or more of the hydroxyl groups is replaced with halogen, aliphatic groups, or functionalized as ethers or amines. In one embodiment, the 2'-position of the furanose residue is substituted by any of an O-methyl, O-alkyl, O-allyl, S-alkyl, S-allyl, or halo group. Methods of synthesis of 2'-modified sugars are described, e.g., in Sproat, et al., Nucl. Acid Res. 19:733-738 (1991); Cotten, et al., Nucl. Acid Res. 19:2629-2635 (1991); and Hobbs, et al., Biochemistry 12:5138-5145 (1973). Other modifications are known to one of ordinary skill in the art. Such modifications may be pre-SELEX™ process modifications or post-SELEX™ process modifications (modification of previously identified unmodified ligands) or may be made by incorporation into the SELEX™ process.

Pre- SELEX™ process modifications or those made by incorporation into the SELEX™ process yield nucleic acid ligands with both specificity for their SELEX™ target and improved stability, *e.g., in vivo* stability. Post-SELEX™ process modifications made to nucleic acid ligands may result in improved stability, *e.g., in vivo* stability without adversely affecting the binding capacity of the nucleic acid ligand.

The SELEX™ method encompasses combining selected oligonucleotides with other selected oligonucleotides and non-oligonucleotide functional units as described in U.S. Patent No. 5,637,459 and U.S. Patent No. 5,683,867. The SELEX™ method further encompasses combining selected nucleic acid ligands with lipophilic or non-immunogenic high molecular weight compounds in a diagnostic or therapeutic complex, as described, e.g., in U.S. Patent No. 6,011,020, U.S. Patent No. 6,051,698, and PCT Publication No. WO 98/18480. These patents and applications teach the combination of a broad array of shapes and other properties, with the efficient amplification and replication properties of oligonucleotides, and with the desirable properties of other molecules.

The identification of nucleic acid ligands to small, flexible peptides via the SELEX™ method has also been explored. Small peptides have flexible structures and usually exist in solution in an equilibrium of multiple conformers, and thus it was initially thought that binding affinities may be limited by the conformational entropy lost upon binding a flexible peptide. However, the feasibility of identifying nucleic acid ligands to small peptides in solution was demonstrated in U.S. Patent No. 5,648,214. In this patent, high affinity RNA nucleic acid ligands to substance P, an 11 amino acid peptide, were identified.

The aptamers with specificity and binding affinity to the target(s) of the present invention are typically selected by the SELEX™ process as described herein. As part of the SELEX™ process, the sequences selected to bind to the target are then optionally minimized to determine the minimal sequence having the desired binding affinity. The selected sequences and/or the minimized sequences are optionally optimized by performing random or directed mutagenesis of the sequence to increase binding affinity or alternatively to determine which positions in the sequence are essential for binding activity. Additionally, selections can be performed with sequences incorporating modified nucleotides to stabilize the aptamer molecules against degradation *in vivo.*

### 2' Modified SELEX™

In order for an aptamer to be suitable for use as a therapeutic, it is preferably inexpensive to synthesize, safe and stable *in vivo.* Wild-type RNA and DNA aptamers are typically not stable *in vivo* because of their susceptibility to degradation by nucleases. Resistance to nuclease degradation can be greatly increased by the incorporation of modifying groups at the 2'-position. Fluoro and amino groups have been successfully incorporated into oligonucleotide pools from which aptamers have been subsequently selected. However, these modifications greatly increase the cost of synthesis of the resultant aptamer, and may introduce safety concerns in some cases because of the possibility that the modified nucleotides could be recycled into host DNA by degradation of the modified oligonucleotides and subsequent use of the nucleotides as substrates for DNA synthesis.

Aptamers that contain 2'-O-methyl ("2'-OMe") nucleotides, as provided herein, overcome many of these drawbacks. Oligonucleotides containing 2'-OMe nucleotides are nuclease-resistant and inexpensive to synthesize. Although 2'-OMe nucleotides are ubiquitous in biological systems, natural polymerases do not accept 2'-OMe NTPs as substrates under physiological conditions, thus there are no safety concerns over the recycling of 2'-OMe nucleotides into host DNA.

The SELEX™ method used to generate 2'-modified aptamers is described, e.g., in U.S. Provisional Patent Application Serial No. 60/430,761, filed December 3, 2002, U.S. Provisional Patent Application Serial No. 60/487,474, filed July 15, 2003, U.S. Provisional Patent Application Serial No. 60/517,039, filed November 4, 2003, U.S. Patent Application No. 10/729,581, filed December 3, 2003, and U.S. Patent Application No. 10/873,856, filed June 21, 2004, entitled "Method *for in vitro* Selection of 2'-O-methyl Substituted Nucleic Acids".

The present invention also provides materials and methods to produce stabilized oligonucleotides, including, *e.g.,* aptamers, that contain modified nucleotides *(e.g.,* nucleotides which have a modification at the 2'position) which make the oligonucleotide more stable than the unmodified oligonucleotide. The stabilized oligonucleotides produced by the materials and methods of the present invention are also more stable to enzymatic and chemical degradation as well as thermal and physical degradation. For example, oligonucleotides containing 2'-O-methyl nucleotides are nuclease-resistant and inexpensive to synthesize. Although 2'-O-methyl nucleotides are ubiquitous in biological systems, natural polymerases do not accept 2'-O-methyl NTPs as substrates under physiological conditions, thus there are no safety concerns over the recycling of 2'-O-methyl nucleotides into host DNA.

Although there are several examples of 2'-OMe containing aptamers in the literature (see, e.g., Green et al., Current Biology 2, 683-695, 1995) these were generated by the in *vitro* selection of libraries of modified transcripts in which the C and U residues were 2'-fluoro (2'-F) substituted and the A and G residues were 2'-OH, Once functional sequences were identified then each A and G residue was tested for tolerance to 2'-OMe substitution, and the aptamer was re-synthesized having all A and G residues which tolerated 2'-OMe substitution as 2'-OMe residues. Most of the A and G residues of aptamers generated in this two-step fashion tolerate substitution with 2'-OMe residues, although, on average, approximately 20% do not. Consequently, aptamers generated using this method tend to contain from two to four 2'-OH residues, and stability and cost of synthesis are compromised as a result. By incorporating modified nucleotides into the transcription reaction which generate stabilized oligonucleotides used in oligonucleotide pools from which aptamers are selected and enriched by SELEX™ (and/or any of its variations and improvements, including those described herein), the methods of the present invention eliminate the need for stabilizing the selected aptamer oligonucleotides *(e.g.,* by resynthesizing the aptamer oligonucleotides with modified nucleotides).

In one embodiment, the present invention provides aptamers comprising combinations of 2'-OH, 2'-F, 2'-deoxy, and 2'-OMe modifications of the ATP, GTP, CTP, TTP, and UTP nucleotides. In another embodiment, the present invention provides aptamers comprising combinations of 2'-OH, 2'-F, 2'-deoxy, 2'-OMe, 2'-NH2, and 2'-methoxyethyl modifications of the ATP, GTP, CTP, TTP, and UTP nucleotides. In another embodiment, the present invention provides aptamers comprising 56 combinations of 2'-OH, 2'-F, 2'-deoxy, 2'-OMe, 2'-NH2, and 2'-methoxyethyl modifications of the ATP, GTP, CTP, TTP, and UTP nucleotides.

2' modified aptamers of the invention are created using modified polymerases, e.g., a modified T7 polymerase, having a rate of incorporation of modified nucleotides having bulky substituents at the furanose 2' position that is higher than that of wild-type polymerases. For example, a single mutant T7 polymerase (Y639F) in which the tyrosine residue at position 639 has been changed to phenylalanine readily utilizes 2'deoxy, 2'amino-, and 2'fluoro-nucleotide triphosphates (NTPs) as substrates and has been widely used to synthesize modified RNAs for a variety of applications. However, this mutant T7 polymerase reportedly can not readily utilize (i.e., incorporate) NTPs with bulky 2'-substituents such as 2'-OMe or 2'-azido (2'-N3) substituents. For incorporation of bulky 2' substituents, a double T7 polymerase mutant (Y639F/H784A) having the histidine at position 784 changed to an alanine residue in addition to the Y639F mutation has been described and has been used in limited circumstances to incorporate modified pyrimidine NTPs. See Padilla, R. and Sousa, R., Nucleic Acids Res., 2002, 30(24): 138. A single mutant T7 polymerase (H784A) having the histidine at position 784 changed to an alanine residue has also been described. Padilla et al., Nucleic Acids Research, 2002, 30: 138. In both the Y639F/H784A double mutant and H784A single mutant T7 polymerases, the change to a smaller amino acid residue such as alanine allows for the incorporation of bulkier nucleotide substrates, e.g., 2'-OMe substituted nucleotides.

Generally, it has been found that under the conditions disclosed herein, the Y693F single mutant can be used for the incorporation of all 2'-OMe substituted NTPs except GTP and the Y639F/H784A double mutant can be used for the incorporation of all 2'-OMe substituted NTPs including GTP. It is expected that the H784A single mutant possesses properties similar to the Y639F and the Y639F/H784A mutants when used under the conditions disclosed herein.

2'-modified oligonucleotides may be synthesized entirely of modified nucleotides, or with a subset of modified nucleotides. The modifications can be the same or different. All nucleotides may be modified, and all may contain the same modification. All nucleotides may be modified, but contain different modifications, *e.g.,* all nucleotides containing the same base may have one type of modification, while nucleotides containing other bases may have different types of modification. All purine nucleotides may have one type of modification (or are unmodified), while all pyrimidine nucleotides have another, different type of modification (or are unmodified). In this way, transcripts, or pools of transcripts are generated using any combination of modifications, including for example, ribonucleotides (2'-OH), deoxyribonucleotides (2'-deoxy), 2'-F, and 2'-OMe nucleotides. A transcription mixture containing 2'-OMe C and U and 2'-OH A and G is referred to as an "rRmY" mixture and aptamers selected therefrom are referred to as "rRmY" aptamers. A transcription mixture containing deoxy A and G and 2'-OMe U and C is referred to as a "dRmY" mixture and aptamers selected therefrom are referred to as "dRmY" aptamers. A transcription mixture containing 2'-OMe A, C, and U, and 2'-OH G is referred to as a "rGmH" mixture and aptamers selected therefrom are referred to as "rGmH" aptamers. A transcription mixture alternately containing 2'-OMe A, C, U and G and 2'-OMe A, U and C and 2'-F G is referred to as an "alternating mixture" and aptamers selected therefrom are referred to as "alternating mixture" aptamers. A transcription mixture containing 2'-OMe A, U, C, and G, where up to 10% of the G's are ribonucleotides is referred to as a "r/mGmH" mixture and aptamers selected therefrom are referred to as "r/mGmH" aptamers. A transcription mixture containing 2'-OMe A, U, and C, and 2'-F G is referred to as a "fGmH" mixture and aptamers selected therefrom are referred to as "fGmH" aptamers. A transcription mixture containing 2'-OMe A, U, and C, and deoxy G is referred to as a "dGmH" mixture and aptamers selected therefrom are referred to as "dGmH" aptamers. A transcription mixture containing deoxy A, and 2'-OMe C, G and U is referred to as a "dAmB" mixture and aptamers selected therefrom are referred to as "dAmB" aptamers, and a transcription mixture containing all 2'-OH nucleotides is referred to as a "rN" mixture and aptamers selected therefrom are referred to as "rN" or "rRrY" aptamers. A "mRmY" aptamer is one containing all 2'-O-methyl nucleotides and is usually derived from a r/mGmH oligonucleotide by post-SELEX™ replacement, when possible, of any 2'-OH Gs with 2'-OMe Gs.

A preferred embodiment includes any combination of 2'-OH, 2'-deoxy and 2'-OMe nucleotides. A more preferred embodiment includes any combination of 2'-deoxy and 2'-OMe nucleotides. An even more preferred embodiment is with any combination of 2'-deoxy and 2'-OMe nucleotides in which the pyrimidines are 2'-OMe (such as dRmY, mRmY or dGmH).

Incorporation of modified nucleotides into the aptamers of the invention is accomplished before (pre-) the selection process *(e.g.,* a pre-SELEX™ process modification). Optionally, aptamers of the invention in which modified nucleotides have been incorporated by pre-SELEX™ process modification can be further modified by post-SELEX™ process modification (*i.e*., a post-SELEX™ process modification after a pre-SELEX™ modification). Pre-SELEX™ process modifications yield modified nucleic acid ligands with specificity for the SELEX™ target and also improved *in vivo* stability. Post-SELEX™ process modifications, *i.e.,* modification *(e.g.,* truncation, deletion, substitution or additional nucleotide modifications of previously identified ligands having nucleotides incorporated by pre-SELEX™ process modification) can result in a further improvement of *in vivo* stability without adversely affecting the binding capacity of the nucleic acid ligand having nucleotides incorporated by pre-SELEX™ process modification.

To generate pools of 2'-modified *(e.g.,* 2'-OMe) RNA transcripts in conditions under which a polymerase accepts 2'-modified NTPs the preferred polymerase is the Y693F/H784A double mutant or the Y693F single mutant. Other polymerases, particularly those that exhibit a high tolerance for bulky 2'-substituents, may also be used in the present invention. Such polymerases can be screened for this capability by assaying their ability to incorporate modified nucleotides under the transcription conditions disclosed herein.

A number of factors have been determined to be important for the transcription conditions useful in the methods disclosed herein. For example, increases in the yields of modified transcript are observed when a leader sequence is incorporated into the 5' end of a fixed sequence at the 5' end of the DNA transcription template, such that at least about the first 6 residues of the resultant transcript are all purines.

Another important factor in obtaining transcripts incorporating modified nucleotides is the presence or concentration of 2'-OH GTP. Transcription can be divided into two phases: the first phase is initiation, during which an NTP is added to the 3'-hydroxyl end of GTP (or another substituted guanosine) to yield a dinucleotide which is then extended by about 10-12 nucleotides, the second phase is elongation, during which transcription proceeds beyond the addition of the first about 10-12 nucleotides. It has been found that small amounts of 2'-OH GTP added to a transcription mixture containing an excess of 2'-OMe GTP are sufficient to enable the polymerase to initiate transcription using 2'-OH GTP, but once transcription enters the elongation phase the reduced discrimination between 2'-OMe and 2'-OH GTP, and the excess of 2'-OMe GTP over 2'-OH GTP allows the incorporation of principally the 2'-OMe GTP.

Another important factor in the incorporation of 2'-OMe substituted nucleotides into transcripts is the use of both divalent magnesium and manganese in the transcription mixture. Different combinations of concentrations of magnesium chloride and manganese chloride have been found to affect yields of 2'-O-methylated transcripts, the optimum concentration of the magnesium and manganese chloride being dependent on the concentration in the transcription reaction mixture ofNTPs which complex divalent metal ions. To obtain the greatest yields of maximally 2' substituted O-methylated transcript (*i.e.,* all A, C, and U and about 90% of G nucleotides), concentrations of approximately 5 mM magnesium chloride and 1.5 mM manganese chloride are preferred when each NTP is present at a concentration of 0.5 mM. When the concentration of each NTP is 1.0 mM, concentrations of approximately 6.5 mM magnesium chloride and 2.0 mM manganese chloride are preferred. When the concentration of each NTP is 2.0 mM, concentrations of approximately 9.6 mM magnesium chloride and 2.9 mM manganese chloride are preferred. In any case, departures from these concentrations of up to two-fold still give significant amounts of modified transcripts.

Priming transcription with GMP or guanosine is also important. This effect results from the specificity of the polymerase for the initiating nucleotide. As a result, the 5'-terminal nucleotide of any transcript generated in this fashion is likely to be 2'-OH G. The preferred concentration of GMP (or guanosine) is 0.5 mM and even more preferably 1 mM. It has also been found that including PEG, preferably PEG-8000, in the transcription reaction is useful to maximize incorporation of modified nucleotides.

For maximum incorporation of 2'-OMe ATP (100%), UTP(100%), CTP(100%) and GTP (∼90%) ("r/mGmH") into transcripts the following conditions are preferred: HEPES buffer 200 mM, DTT 40 mM, spermidine 2 mM, PEG-8000 10% (w/v), Triton X-100 0.01% (w/v), MgCl₂ 5 mM (6.5 mM where the concentration of each 2'-OMe NTP is 1.0 mM), MnCl₂ 1.5 mM (2.0 mM where the concentration of each 2'-OMe NTP is 1.0 mM), 2'-OMe NTP (each) 500 µM (more preferably, 1.0 mM), 2'-OH GTP 30 µM, 2'-OH·GMP 500 µM, pH 7.5, Y639F/H784A T7 RNA Polymerase 15 units/ml, inorganic pyrophosphatase 5 units/ml, and an all-purine leader sequence of at least 8 nucleotides long. As used herein, one unit of the Y639F/H784A mutant T7 RNA polymerase, or any other mutant T7 RNA polymerase specified herein) is defined as the amount of enzyme required to incorporate 1 nmole of 2'-OMe NTPs into transcripts under the r/mGmH conditions. As used herein, one unit of inorganic pyrophosphatase is defined as the amount of enzyme that will liberate 1.0 mole of inorganic orthophosphate per minute at pH 7.2 and 25 °C.

For maximum incorporation (100%) of 2'-OMe ATP, UTP and CTP ("rGmH") into transcripts the following conditions are preferred: HEPES buffer 200 mM, DTT 40 mM, spermidine 2 mM, PEG-8000 10% (w/v), Triton X-100 0.01% (w/v), MgCl₂ 5 mM (9.6 mM, where the concentration of each 2'-OMe NTP is 2.0 mM), MnCl₂ 1.5 mM (2.9 mM where the concentration of each 2'-OMe NTP is 2.0 mM), 2'-OMe NTP (each) 500 µM (more preferably, 2.0 mM), pH 7.5, Y639F T7 RNA Polymerase 15 units/ml, inorganic pyrophosphatase 5 units/ml, and an all-purine leader sequence of at least 8 nucleotides long.

For maximum incorporation (100%) of 2'-OMe UTP and CTP ("rRmY") into transcripts the following conditions are preferred: HEPES buffer 200 mM, DTT 40 mM, spermidine 2 mM, PEG-8000 10% (w/v), Triton X-100 0.01% (w/v), MgCl₂ 5 mM (9.6 mM where the concentration of each 2'-OMe NTP is 2.0 mM), MnCl₂ 1.5 mM (2.9 mM where the concentration of each 2'-OMe NTP is 2.0 mM), 2'-OMe NTP (each) 500µM (more preferably, 2.0 mM), pH 7.5, Y639F/H784A T7 RNA Polymerase 15 units/ml, inorganic pyrophosphatase 5 units/ml, and an all-purine leader sequence of at least 8 nucleotides long.

For maximum incorporation (100%) of deoxy ATP and GTP and 2'-OMe UTP and CTP ("dRmY") into transcripts the following conditions are preferred: HEPES buffer 200 mM, DTT 40 mM, spermine 2 mM, spermidine 2 mM, PEG-8000 10% (w/v), Triton X-100 0.01% (w/v), MgCl2 9.6 mM, MnCl2 2.9 mM, 2'-OMe NTP (each) 2.0 mM, pH 7.5, Y639F T7 RNA Polymerase 15 units/mL, inorganic pyrophosphatase 5 units/mL, and an all-purine leader sequence of at least 8 nucleotides long.

For maximum incorporation (100%) of 2'-OMe ATP, UTP and CTP and 2'-F GTP ("fGmH") into transcripts the following conditions are preferred: HEPES buffer 200 mM, DTT 40 mM, spermidine 2 mM, PEG-8000 10% (w/v), Triton X-100 0.01 % (w/v), MgCl₂ 9.6 mM, MnCl₂ 2.9 mM, 2'-OMe NTP (each) 2.0 mM, pH 7.5, Y639F T7 RNA Polymerase 15 units/ml, inorganic pyrophosphatase 5 units/ml, and an all-purine leader sequence of at least 8 nucleotides long.

For maximum incorporation (100%) of deoxy ATP and 2'-OMe UTP, GTP and CTP ("dAmB") into transcripts the following conditions are preferred: HEPES buffer 200 mM, DTT 40 mM, spermidine 2 mM, PEG-8000 10% (w/v), Triton X-100 0.01% (w/v), MgCl₂ 9.6 mM, MnCl₂ 2.9 mM, 2'-OMe NTP (each) 2.0 mM, pH 7.5, Y639F T7 RNA Polymerase 15 units/ml, inorganic pyrophosphatase 5 units/ml, and an all-purine leader sequence of at least 8 nucleotides long.

For each of the above (a) transcription is preferably performed at a temperature of from about 20 °C to about 50 °C, preferably from about 30 °C to 45 °C, and more preferably at about 37 °C for a period of at least two hours and (b) 50-300 nM of a double stranded DNA transcription template is used (200 nM template is used in round 1 to increase diversity (300 nM template is used in dRmY transcriptions)), and for subsequent rounds approximately 50 nM, a 1/10 dilution of an optimized PCR reaction, using conditions described herein, is used). The preferred DNA transcription templates are described below (where ARC254 and ARC256 transcribe under all 2'-OMe conditions and ARC255 transcribes under rRmY conditions).

### ARC254:

5’-CATCGATGCTAGTCGTAACGATCCNNNNNNNNNNNNNNNNNNNNNNNN
NNNNNNCGAGAACGTTCTCTCCTCTCCCTATAGTGAGTCGTATTA-3’ (SEQ
ID NO:171)

### ARC255:

5’-CATGCATCGCGACTGACTAGCCGNNNNNNNNNNNNNNNNNNNNNNNN
NNNNNNGTAGAACGTTCTCTCCTCTCCCTATAGTGAGTCGTATTA-3’ (SEQ
ID NO:172)

### ARC256:

5'-CATCGATCGATCGATCGACAGCGNNNNNNNNNNNNNNNNNNNNNNNN
NNNNNNGTAGAACGTTCTCTCCTCTCCCTATAGTGAGTCGTATTA–3' (SEQ
ID NO:173)

Under rN transcription conditions of the present invention, the transcription reaction mixture comprises 2'-OH adenosine triphosphates (ATP), 2'-OH guanosine triphosphates (GTP), 2'-OH cytidine triphosphates (CTP), and 2'-OH uridine triphosphates (UTP). The modified oligonucleotides produced using the rN transcription mixtures of the present invention comprise substantially all 2'-OH adenosine, 2'-OH guanosine, 2'-OH cytidine, and 2'-OH uridine. In a preferred embodiment of rN transcription, the resulting modified oligonucleotides comprise a sequence where at least 80% of all adenosine nucleotides are 2'-OH adenosine, at least 80% of all guanosine nucleotides are 2'-OH guanosine, at least 80% of all cytidine nucleotides are 2'-OH cytidine, and at least 80% of all uridine nucleotides are 2'-OH uridine. In a more preferred embodiment of rN transcription, the resulting modified oligonucleotides of the present invention comprise a sequence where at least 90% of all adenosine nucleotides are 2'-OH adenosine, at least 90% of all guanosine nucleotides are 2'-OH guanosine, at least 90% of all cytidine nucleotides are 2'-OH cytidine, and at least 90% of all uridine nucleotides are 2'-OH uridine. In a most preferred embodiment of rN transcription, the modified oligonucleotides of the present invention comprise a sequence where 100% of all adenosine nucleotides are 2'-OH adenosine, 100% of all guanosine nucleotides are 2'-OH guanosine, 100% of all cytidine nucleotides are 2'-OH cytidine, and 100% of all uridine nucleotides are 2'-OH uridine.

Under rRmY transcription conditions of the present invention, the transcription reaction mixture comprises 2'-OH adenosine triphosphates, 2'-OH guanosine triphosphates, 2'-O-methyl cytidine triphosphates, and 2'-O-methyl uridine triphosphates. The modified oligonucleotides produced using the rRmY transcription mixtures of the present invention comprise substantially all 2'-OH adenosine, 2'-OH guanosine, 2'-O-methyl cytidine and 2'-O-methyl uridine. In a preferred embodiment, the resulting modified oligonucleotides comprise a sequence where at least 80% of all adenosine nucleotides are 2'-OH adenosine, at least 80% of all guanosine nucleotides are 2'-OH guanosine, at least 80% of all cytidine nucleotides are 2'-O-methyl cytidine and at least 80% of all uridine nucleotides are 2'-O-methyl uridine. In a more preferred embodiment, the resulting modified oligonucleotides comprise a sequence where at least 90% of all adenosine nucleotides are 2'-OH adenosine, at least 90% of all guanosine nucleotides are 2'-OH guanosine, at least 90% of all cytidine nucleotides are 2'-O-methyl cytidine and at least 90% of all uridine nucleotides are 2'-O-methyl uridine In a most preferred embodiment, the resulting modified oligonucleotides comprise a sequence where 100% of all adenosine nucleotides are 2'-OH adenosine, 100% of all guanosine nucleotides are 2'-OH guanosine, 100% of all cytidine nucleotides are 2'-O-methyl cytidine and 100% of all uridine nucleotides are 2'-O-methyl uridine.

Under dRmY transcription conditions of the present invention, the transcription reaction mixture comprises 2'-deoxy adenosine triphosphates, 2'-deoxy guanosine triphosphates, 2'-O-methyl cytidine triphosphates, and 2'-O-methyl uridine triphosphates. The modified oligonucleotides produced using the dRmY transcription conditions of the present invention comprise substantially all 2'-deoxy adenosine, 2'-deoxy guanosine, 2'-O-methyl cytidine, and 2'-O-methyl uridine. In a preferred embodiment, the resulting modified oligonucleotides of the present invention comprise a sequence where at least 80% of all adenosine nucleotides are 2'-deoxy adenosine, at least 80% of all guanosine nucleotides are 2'-deoxy guanosine, at least 80% of all cytidine nucleotides are 2'-O-methyl cytidine, and at least 80% of all uridine nucleotides are 2'-O-methyl uridine. In a more preferred embodiment, the resulting modified oligonucleotides of the present invention comprise a sequence where at least 90% of all adenosine nucleotides are 2'-deoxy adenosine, at least 90 % of all guanosine nucleotides are 2'-deoxy guanosine, at least 90% of all cytidine nucleotides are 2'-O-methyl cytidine, and at least 90% of all uridine nucleotides are 2'-O-methyl uridine. In a most preferred embodiment, the resulting modified oligonucleotides of the present invention comprise a sequence where 100% of all adenosine nucleotides are 2'-deoxy adenosine, 100% of all guanosine nucleotides are 2'-deoxy guanosine, 100% of all cytidine nucleotides are 2'-O-methyl cytidine, and 100% of all uridine nucleotides are 2'-O-methyl uridine.

Under rGmH transcription conditions of the present invention, the transcription reaction mixture comprises 2'-OH guanosine triphosphates, 2'-O-methyl cytidine triphosphates, 2'-O-methyl uridine triphosphates, and 2'-O-methyl adenosine triphosphates. The modified oligonucleotides produced using the rGmH transcription mixtures of the present invention comprise substantially all 2'-OH guanosine, 2'-O-methyl cytidine, 2'-O-methyl uridine, and 2'-O-methyl adenosine. In a preferred embodiment, the resulting modified oligonucleotides comprise a sequence where at least 80% of all guanosine nucleotides are 2'-OH guanosine, at least 80% of all cytidine nucleotides are 2'-O-methyl cytidine, at least 80% of all uridine nucleotides are 2'-O-methyl uridine, and at least 80% of all adenosine nucleotides are 2'-O-methyl adenosine. In a more preferred embodiment, the resulting modified oligonucleotides comprise a sequence where at least 90% of all guanosine nucleotides are 2'-OH guanosine, at least 90% of all cytidine nucleotides are 2'-O-methyl cytidine, at least 90% of all uridine nucleotides are 2'-O-methyl uridine, and at least 90% of all adenosine nucleotides are 2'-O-methyl adenosine. In a most preferred embodiment, the resulting modified oligonucleotides comprise a sequence where 100% of all guanosine nucleotides are 2'-OH guanosine, 100% of all cytidine nucleotides are 2'-O-methyl cytidine, 100% of all uridine nucleotides are 2'-O-methyl uridine, and 100% of all adenosine nucleotides are 2'-O-methyl adenosine.

Under r/mGmH transcription conditions of the present invention, the transcription reaction mixture comprises 2'-O-methyl adenosine triphosphate, 2'-O-methyl cytidine triphosphate, 2'-O-methyl guanosine triphosphate, 2'-O-methyl uridine triphosphate and 2'-OH guanosine triphosphate. The resulting modified oligonucleotides produced using the r/mGmH transcription mixtures of the present invention comprise substantially all 2'-O-methyl adenosine, 2'-O-methyl cytidine, 2'-O-methyl guanosine, and 2'-O-methyl uridine, wherein the population of guanosine nucleotides has a maximum of about 10% 2'-OH guanosine. In a preferred embodiment, the resulting r/mGmH modified oligonucleotides of the present invention comprise a sequence where at least 80% of all adenosine nucleotides are 2'-O-methyl adenosine, at least 80% of all cytidine nucleotides are 2'-O-methyl cytidine, at least 80% of all guanosine nucleotides are 2'-O-methyl guanosine, at least 80% of all uridine nucleotides are 2'-O-methyl uridine, and no more than about 10% of all guanosine nucleotides are 2'-OH guanosine: In a more preferred embodiment, the resulting modified oligonucleotides comprise a sequence where at least 90% of all adenosine nucleotides are 2'-O-methyl adenosine, at least 90% of all cytidine nucleotides are 2'-O-methyl cytidine, at least 90% of all guanosine nucleotides are 2'-O-methyl guanosine, at least 90% of all uridine nucleotides are 2'-O-methyl uridine, and no more than about 10% of all guanosine nucleotides are 2'-OH guanosine. In a most preferred embodiment, the resulting modified oligonucleotides comprise a sequence where 100% of all adenosine nucleotides are 2'-O-methyl adenosine, 100% of all cytidine nucleotides are 2'-O-methyl cytidine, 90% of all guanosine nucleotides are 2'-O-methyl guanosine, and 100% of all uridine nucleotides are 2'-O-methyl uridine, and no more than about 10% of all guanosine nucleotides are 2'-OH guanosine.

Under fGmH transcription conditions of the present invention, the transcription reaction mixture comprises 2'-O-methyl adenosine triphosphates, 2'-O-methyl uridine triphosphates, 2'-O-methyl cytidine triphosphates, and 2'-F guanosine triphosphates. The modified oligonucleotides produced using the fGmH transcription conditions of the present invention comprise substantially all 2'-O-methyl adenosine, 2'-O-methyl uridine, 2'-O-methyl cytidine, and 2'-F guanosine. In a preferred embodiment, the resulting modified oligonucleotides comprise a sequence where at least 80% of all adenosine nucleotides are 2'-O-methyl adenosine, at least 80% of all uridine nucleotides are 2'-O-methyl uridine, at least 80% of all cytidine nucleotides are 2'-O-methyl cytidine, and at least 80% of all guanosine nucleotides are 2'-F guanosine. In a more preferred embodiment, the resulting modified oligonucleotides comprise a sequence where at least 90% of all adenosine nucleotides are 2'-O-methyl adenosine, at least 90% of all uridine nucleotides are 2'-O-methyl uridine, at least 90% of all cytidine nucleotides are 2'-O-methyl cytidine, and at least 90% of all guanosine nucleotides are 2'-F guanosine. In a most preferred embodiment, the resulting modified oligonucleotides comprise a sequence where 100% of all adenosine nucleotides are 2'-O-methyl adenosine, 100% of all uridine nucleotides are 2'-O-methyl uridine, 100% of all cytidine nucleotides are 2'-O-methyl cytidine, and 100% of all guanosine nucleotides are 2'-F guanosine.

Under dAmB transcription conditions of the present invention, the transcription reaction mixture comprises 2'-deoxy adenosine triphosphates, 2'-O-methyl cytidine triphosphates, 2'-O-methyl guanosine triphosphates, and 2'-O-methyl uridine triphosphates. The modified oligonucleotides produced using the dAmB transcription mixtures of the present invention comprise substantially all 2'-deoxy adenosine, 2'-O-methyl cytidine, 2'-O-methyl guanosine, and 2'-O-methyl uridine. In a preferred embodiment, the resulting modified oligonucleotides comprise a sequence where at least 80% of all adenosine nucleotides are 2'-deoxy adenosine, at least 80% of all cytidine nucleotides are 2'-O-methyl cytidine, at least 80% of all guanosine nucleotides are 2'-O-methyl guanosine, and at least 80% of all uridine nucleotides are 2'-O-methyl uridine. In a more preferred embodiment, the resulting modified oligonucleotides comprise a sequence where at least 90% of all adenosine nucleotides are 2'-deoxy adenosine, at least 90% of all cytidine nucleotides are 2'-O-methyl cytidine, at least 90% of all guanosine nucleotides are 2'-O-methyl guanosine, and at least 90% of all uridine nucleotides are 2'-O-methyl uridine. In a most preferred embodiment, the resulting modified oligonucleotides of the present invention comprise a sequence where 100% of all adenosine nucleotides are 2'-deoxy adenosine, 100% of all cytidine nucleotides are 2'-O-methyl cytidine, 100% of all guanosine nucleotides are 2'-O-methyl guanosine, and 100% of all uridine nucleotides are 2'-O-methyl uridine.

In each case, the transcription products can then be used as the library in the SELEX™ process to identify aptamers and/or to determine a conserved motif of sequences that have binding specificity to a given target. The resulting sequences are already partially stabilized, eliminating this step from the process to arrive at an optimized aptamer sequence and giving a more highly stabilized aptamer as a result. Another advantage of the 2'-OMe SELEX™ process is that the resulting sequences are likely to have fewer 2'-OH nucleotides required in the sequence, possibly none. To the extent 2'OH nucleotides remain they can be removed by performing post-SELEX™ modifications.

As described below, lower but still useful yields of transcripts fully incorporating 2' substituted nucleotides can be obtained under conditions other than the optimized conditions described above. For example, variations to the above transcription conditions include:

The HEPES buffer concentration can range from 0 to 1 M. The present invention also contemplates the use of other buffering agents having a pKa between 5 and 10 including, for example, Tris-hydroxymethyl-aminomethane.

The DTT concentration can range from 0 to 400 mM. The methods of the present invention also provide for the use of other reducing agents including, for example, mercaptoethanol.

The spermidine and/or spermine concentration can range from 0 to 20 mM.

The PEG-8000 concentration can range from 0 to 50 % (w/v). The methods of the present invention also provide for the use of other hydrophilic polymer including, for example, other molecular weight PEG or other polyalkylene glycols.

The Triton X-100 concentration can range from 0 to 0.1 % (w/v). The methods of the present invention also provide for the use of other non-ionic detergents including, for example, other detergents, including other Triton-X detergents.

The MgCl2 concentration can range from 0.5 mM to 50 mM. The MnCl2 concentration can range from 0.15 mM to 15 mM. Both MgCl2 and MnCl2 must be present within the ranges described and in a preferred embodiment are present in about a 10 to about 3 ratio of MgCl2:MnCl2, preferably, the ratio is about 3-5:1, more preferably, the ratio is about 3-4:1.

The 2'-OMe NTP concentration (each NTP) can range from 5 µM to 5 mM.

The 2'-OH GTP concentration can range from 0 µM to 300 µM.

The 2'-OH GMP concentration can range from 0 to 5 mM.

The pH can range from pH 6 to pH 9. The methods of the present invention can be practiced within the pH range of activity of most polymerases that incorporate modified nucleotides. In addition, the methods of the present invention provide for the optional use of chelating agents in the transcription reaction condition including, for example, EDTA, EGTA, and DTT.

In some embodiments aptamer therapeutics of the present invention have great affinity and specificity to their targets while reducing the deleterious side effects from non-naturally occurring nucleotide substitutions if the aptamer therapeutics break down in the body of patients or subjects. In some embodiments, the therapeutic compositions containing the aptamer therapeutics of the present invention are free of or have a reduced amount of fluorinated nucleotides.

The aptamers of the present invention can be synthesized using any oligonucleotide synthesis techniques known in the art including solid phase oligonucleotide synthesis techniques well known in the art (see, e.g., Froehler et al., Nucl. Acid Res. 14:5399-5467 (1986) and Froehler et al., Tet. Lett. 27:5575-5578 (1986)) and solution phase methods such as triester synthesis methods (see, e.g., Sood et al., Nucl. Acid Res. 4:2557 (1977) and Hirose et al., Tet. Lett., 28:2449 (1978)).

### Aptamers Having Immunostimulatory Motifs

Recognition of bacterial DNA by the vertebrate immune system is based on the recognition of unmethylated CG dinucleotides in particular sequence contexts ("CpG motifs"). One receptor that recognizes such a motif is Toll-like receptor 9 ("TLR 9"), a member of a family of Toll-like receptors (∼10 members) that participate in the innate immune response by recognizing distinct microbial components. TLR 9 binds unmethylated oligodeoxynucleotide ("ODN") CpG sequences in a sequence-specific manner. The recognition of CpG motifs triggers defense mechanisms leading to innate and ultimately acquired immune responses. For example, activation of TLR 9 in mice induces activation of antigen presenting cells, up regulation of MHC class I and II molecules and expression of important co-stimulatory molecules and cytokines including IL-12 and IL-23. This activation both directly and indirectly enhances B and T cell responses, including robust up regulation of the TH1 cytokine IFN-gamma. Collectively, the response to CpG sequences leads to: protection against infectious diseases, improved immune response to vaccines, an effective response against asthma, and improved antibody-dependent cell-mediated cytotoxicity. Thus, CpG ODNs can provide protection against infectious diseases, function as immuno-adjuvants or cancer therapeutics (monotherapy or in combination with a mAb or other therapies), and can decrease asthma and allergic response.

Aptamers of the present invention comprising one or more CpG or other immunostimulatory sequences can be identified or generated by a variety of strategies using, e.g., the SELEX™ process described herein. The incorporated immunostimulatory sequences can be DNA, RNA and/or a combination DNA/RNA. In general the strategies can be divided into two groups. In group one, the strategies are directed to identifying or generating aptamers comprising both a CpG motif or other immunostimulatory sequence as well as a binding site for a target, where the target (hereinafter "non-CpG target") is a target other than one known to recognize CpG motifs or other immunostimulatory sequences and known to stimulates an immune response upon binding to a CpG motif. The first strategy of this group comprises performing SELEX™ to obtain an aptamer to a specific non-CpG target, preferably a target, where a repressed immune response is relevant to disease development, using an oligonucleotide pool wherein a CpG motif has been incorporated into each member of the pool as, or as part of, a fixed region, *e.g.,* in some embodiments the randomized region of the pool members comprises a fixed region having a CpG motif incorporated therein, and identifying an aptamer comprising a CpG motif. The second strategy of this group comprises performing SELEX™ to obtain an aptamer to a specific non-CpG target preferably a target, where a repressed immune response is relevant to disease development, and following selection appending a CpG motif to the 5' and/or 3' end or engineering a CpG motif into a region, preferably a non-essential region, of the aptamer. The third strategy of this group comprises performing SELEX™ to obtain an aptamer to a specific non-CpG target, preferably a target, where a repressed immune response is relevant to disease development, wherein during synthesis of the pool the molar ratio of the various nucleotides is biased in one or more nucleotide addition steps so that the randomized region of each member of the pool is enriched in CpG motifs, and identifying an aptamer comprising member pool is enriched in CpG motifs, and identifying an aptamer comprising a CpG motif. The fourth strategy of this group comprises performing SELEX™ to obtain an aptamer to a specific non-CpG target, preferably a target, where a repressed immune response is relevant to disease development, and identifying an aptamer comprising a CpG motif. The fifth strategy of this group comprises performing SELEX™ to obtain an aptamer to a specific non-CpG target, preferably a target, where a repressed immune response is relevant to disease development, and identifying an aptamer which, upon binding, stimulates an immune response but which does not comprise a CpG motif.

In group two, the strategies are directed to identifying or generating aptamers comprising a CpG motif and/or other sequences that are bound by the receptors for the CpG motifs (e.g., TLR9 or the other toll-like receptors) and upon binding stimulate an immune response. The first strategy of this group comprises performing SELEX™ to obtain an aptamer to a target known to bind to CpG motifs or other immunostimulatory sequences and upon binding stimulate an immune response using an oligonucleotide pool wherein a CpG motif has been incorporated into each member of the pool as, or as part of, a fixed region, e.g., in some embodiments the randomized region of the pool members comprise a fixed region having a CpG motif incorporated therein, and identifying an aptamer comprising a CpG motif. The second strategy of this group comprises performing SELEX™ to obtain an aptamer to a target known to bind to CpG motifs or other immunostimulatory sequences and upon binding stimulate an immune response and then appending a CpG motif to the 5' and/or 3' end or engineering a CpG motif into a region, preferably a non-essential region, of the aptamer. The third strategy of this group comprises performing SELEX™ to obtain an aptamer to a target known to bind to CpG motifs or other immunostimulatory sequences and upon binding stimulate an immune response wherein during synthesis of the pool, the molar ratio of the various nucleotides is biased in one or more nucleotide addition steps so that the randomized region of each member of the pool is enriched in CpG motifs, and identifying an aptamer comprising a CpG motif. The fourth strategy of this group comprises performing SELEX™ to obtain an aptamer to a target known to bind to CpG motifs or other immunostimulatory sequences and upon binding stimulate an immune response and identifying an aptamer comprising a CpG motif. The fifth strategy of this group comprises performing SELEX™ to obtain an aptamer to a target known to bind to CpG motifs or other immunostimulatory sequences, and identifying an aptamer which upon binding, stimulate an immune response but which does not comprise a CpG motif.

A variety of different classes of CpG motifs have been identified, each resulting upon recognition in a different cascade of events, release of cytokines and other molecules, and activation of certain cell types. See, e.g., CpG Motifs in Bacterial DNA and Their Immune Effects, Annu. Rev. Immunol. 2002, 20:709-760. Additional immunostimulatory motifs are disclosed in the following U.S. Patents: U.S. Patent No. 6,207,646; U.S. Patent No. 6,239,116; U.S. Patent No. 6,429,199; U.S. Patent No. 6,214,806; U.S. Patent No. 6,653,292; U.S. Patent No. 6,426,434; U.S. Patent No. 6,514,948 and U.S. Patent No. 6,498,148. Any of these CpG or other immunostimulatory motifs can be incorporated into an aptamer. The choice of aptamers is dependent on the disease or disorder to be treated. Preferred immunostimulatory motifs are as follows (shown 5' to 3' left to right) wherein "r" designates a purine, "y" designates a pyrimidine, and "X" designates any nucleotide: AACGTTCGAG (SEQ ID NO 52); AACGTT; ACGT, rCGy; rrCGyy, XCGX, XXCGXX, and X1X2CGY1Y2 wherein X1 is G or A, X2 is not C, Y1 is not G and Y2 is preferably T.

In those instances where a CpG motif is incorporated into an aptamer that binds to a specific target other than a target known to bind to CpG motifs and upon binding stimulate an immune response (a "non-CpG target"), the CpG is preferably located in a non-essential region of the aptamer. Non-essential regions of aptamers can be identified by site-directed mutagenesis, deletion analyses and/or substitution analyses. However, any location that does not significantly interfere with the ability of the aptamer to bind to the non-CpG target may be used. In addition to being embedded within the aptamer sequence, the CpG motif may be appended to either or both of the 5' and 3' ends or otherwise attached to the aptamer. Any location or means of attachment may be used so long as the ability of the aptamer to bind to the non-CpG target is not significantly interfered with.

As used herein, "stimulation of an immune response" can mean either (1) the induction of a specific response (e.g., induction of a Th1 response) or of the production of certain molecules or (2) the inhibition or suppression of a specific response (e.g., inhibition or suppression of the Th2 response) or of certain molecules.

### Pharmaceutical Compositions

The invention also includes pharmaceutical compositions containing aptamer molecules. In some embodiments, the compositions are suitable for internal use and include an effective amount of a pharmacologically active compound of the invention, alone or in combination, with one or more pharmaceutically acceptable carriers. The compounds are especially useful in that they have very low, if any toxicity.

Compositions of the invention can be used to treat or prevent a pathology, such as a disease or disorder, or alleviate the symptoms of such disease or disorder in a patient. For example, compositions of the invention can be used to treat or prevent a pathology associated with inflammation. Compositions of the invention are useful for administration to a subject suffering from, or predisposed to, a disease or disorder which is related to or derived from a target to which the aptamers specifically bind.

For example, the target is a protein involved with inflammation, for example, the target protein causes or contributes to inflammation.

Compositions of the invention can be used in a method for treating a patient having a pathology. The method involves administering to the patient a composition comprising aptamers that bind a target (*e.g*., a protein) involved with the pathology, so that binding of the composition to the target alters the biological function of the target, thereby treating the pathology.

The patient having a pathology, *e.g*. the patient treated by the methods of this invention can be a mammal, or more particularly, a human.

In practice, the compounds or their pharmaceutically acceptable salts, are administered in amounts which will be sufficient to exert their desired biological activity.

One aspect of the invention comprises an aptamer composition of the invention in combination with other treatments for inflammatory and autoimmune diseases, cancer, and other related disorders. The aptamer composition of the invention may contain, for example, more than one aptamer. In some examples, an aptamer composition of the invention, containing one or more compounds of the invention, is administered in combination with another useful composition such as an anti-inflammatory agent, an immunosuppressant, an antiviral agent, or the like. Furthermore, the compounds of the invention may be administered in combination with a cytotoxic, cytostatic, or chemotherapeutic agent such as an alkylating agent, anti-metabolite, mitotic inhibitor or cytotoxic antibiotic, as described above. In general, the currently available dosage forms of the known therapeutic agents for use in such combinations will be suitable.

"Combination therapy" (or "co-therapy") includes the administration of an aptamer composition of the invention and at least a second agent as part of a specific treatment regimen intended to provide the beneficial effect from the co-action of these therapeutic agents. The beneficial effect of the combination includes, but is not limited to, pharmacokinetic or pharmacodynamic co-action resulting from the combination of therapeutic agents. Administration of these therapeutic agents in combination typically is carried out over a defined time period (usually minutes, hours, days or weeks depending upon the combination selected).

"Combination therapy" may, but generally is not, intended to encompass the administration of two or more of these therapeutic agents as part of separate monotherapy regimens that incidentally and arbitrarily result in the combinations of the present invention. "Combination therapy" is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single capsule having a fixed ratio of each therapeutic agent or in multiple, single capsules for each of the therapeutic agents.

Sequential or substantially simultaneous administration of each therapeutic agent can be effected by any appropriate route including, but not limited to, topical routes, oral routes, intravenous routes, intramuscular routes, and direct absorption through mucous membrane tissues. The therapeutic agents can be administered by the same route or by different routes. For example, a first therapeutic agent of the combination selected may be administered by injection while the other therapeutic agents of the combination may be administered topically.

Alternatively, for example, all therapeutic agents may be administered topically or all therapeutic agents may be administered by injection. The sequence in which the therapeutic agents are administered is not narrowly critical unless noted otherwise. "Combination therapy" also can embrace the administration of the therapeutic agents as described above in further combination with other biologically active ingredients. Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

Therapeutic or pharmacological compositions of the present invention will generally comprise an effective amount of the active component(s) of the therapy, dissolved or dispersed in a pharmaceutically acceptable medium. Pharmaceutically acceptable media or carriers include any and all solvents, dispersion media, coatings, antibacterial and antifungal agents, isotonic and absorption delaying agents and the like. The use of such media and agents for pharmaceutical active substances is well known in the art. Supplementary active ingredients can also be incorporated into the therapeutic compositions of the present invention.

The preparation of pharmaceutical or pharmacological compositions will be known to those of skill in the art in light of the present disclosure. Typically, such compositions may be prepared as injectables, either as liquid solutions or suspensions; solid forms suitable for solution in, or suspension in, liquid prior to injection; as tablets or other solids for oral administration; as time release capsules; or in any other form currently used, including eye drops, creams, lotions, salves, inhalants and the like. The use of sterile formulations, such as saline-based washes, by surgeons, physicians or health care workers to treat a particular area in the operating field may also be particularly useful. Compositions may also be delivered via microdevice, microparticle or sponge.

Upon formulation, therapeutics will be administered in a manner compatible with the dosage formulation, and in such amount as is pharmacologically effective. The formulations are easily administered in a variety of dosage forms, such as the type of injectable solutions described above, but drug release capsules and the like can also be employed.

In this context, the quantity of active ingredient and volume of composition to be administered depends on the host animal to be treated. Precise amounts of active compound required for administration depend on the judgment of the practitioner and are peculiar to each individual.

A minimal volume of a composition required to disperse the active compounds is typically utilized. Suitable regimes for administration are also variable, but would be typified by initially administering the compound and monitoring the results and then giving further controlled doses at further intervals.

For instance, for oral administration in the form of a tablet or capsule (*e.g*., a gelatin capsule), the active drug component can be combined with an oral, non-toxic, pharmaceutically acceptable inert carrier such as ethanol, glycerol, water and the like. Moreover, when desired or necessary, suitable binders, lubricants, disintegrating agents, and coloring agents can also be incorporated into the mixture. Suitable binders include starch, magnesium aluminum silicate, starch paste, gelatin, methylcellulose, sodium carboxymethylcellulose and/or polyvinylpyrrolidone, natural sugars such as glucose or beta-lactose, corn sweeteners, natural and synthetic gums such as acacia, tragacanth or sodium alginate, polyethylene glycol, waxes, and the like. Lubricants used in these dosage forms include sodium oleate, sodium stearate, magnesium stearate, sodium benzoate, sodium acetate, sodium chloride, silica, talcum, stearic acid, its magnesium or calcium salt and/or polyethyleneglycol, and the like. Disintegrators include, without limitation, starch, methyl cellulose, agar, bentonite, xanthan gum starches, agar, alginic acid or its sodium salt, or effervescent mixtures, and the like. Diluents, include, *e.g*., lactose, dextrose, sucrose, mannitol, sorbitol, cellulose and/or glycine.

The compounds of the invention can also be administered in such oral dosage forms as timed release and sustained release tablets or capsules, pills, powders, granules, elixirs, tinctures, suspensions, syrups and emulsions. Suppositories are advantageously prepared from fatty emulsions or suspensions.

The pharmaceutical compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. The compositions are prepared according to conventional mixing, granulating, or coating methods, and typically contain about 0.1% to 75%, preferably about 1% to 50%, of the active ingredient.

Liquid, particularly injectable compositions can, for example, be prepared by dissolving, dispersing, etc. The active compound is dissolved in or mixed with a pharmaceutically pure solvent such as, for example, water, saline, aqueous dextrose, glycerol, ethanol, and the like, to thereby form the injectable solution or suspension. Additionally, solid forms suitable for dissolving in liquid prior to injection can be formulated.

The compounds of the present invention can be administered in intravenous (both bolus and infusion), intraperitoneal, subcutaneous or intramuscular form, all using forms well known to those of ordinary skill in the pharmaceutical arts. Injectables can be prepared in conventional forms, either as liquid solutions or suspensions.

Parenteral injectable administration is generally used for subcutaneous, intramuscular or intravenous injections and infusions. Additionally, one approach for parenteral administration employs the implantation of a slow-release or sustained-released systems, which assures that a constant level of dosage is maintained, according to U.S. Pat. No. 3,710,795.

Furthermore, preferred compounds for the present invention can be administered in intranasal form via topical use of suitable intranasal vehicles, inhalants, or via transdermal routes, using those forms of transdermal skin patches well known to those of ordinary skill in that art. To be administered in the form of a transdermal delivery system, the dosage administration will, of course, be continuous rather than intermittent throughout the dosage regimen. Other preferred topical preparations include creams, ointments, lotions, aerosol sprays and gels, wherein the concentration of active ingredient would typically range from 0.01 % to 15%, w/w or w/v.

For solid compositions, excipients include pharmaceutical grades of mannitol, lactose, starch, magnesium stearate, sodium saccharin, talcum, cellulose, glucose, sucrose, magnesium carbonate, and the like. The active compound defined above, may be also formulated as suppositories, using for example, polyalkylene glycols, for example, propylene glycol, as the carrier. In some embodiments, suppositories are advantageously prepared from fatty emulsions or suspensions.

The compounds of the present invention can also be administered in the form of liposome delivery systems, such as small unilamellar vesicles, large unilamellar vesicles and multilamellar vesicles. Liposomes can be formed from a variety of phospholipids, containing cholesterol, stearylamine or phosphatidylcholines. In some embodiments, a film of lipid components is hydrated with an aqueous solution of drug to a form lipid layer encapsulating the drug, as described in U.S. Pat. No. 5,262,564. For example, the aptamer molecules described herein can be provided as a complex with a lipophilic compound or non-immunogenic, high molecular weight compound constructed using methods known in the art. An example of nucleic-acid associated complexes is provided in U.S. Patent No. 6,411,020.

The compounds of the present invention may also be coupled with soluble polymers as targetable drug carriers. Such polymers can include polyvinylpyrrolidone, pyran copolymer, polyhydroxypropyl-methacrylamide-phenol, polyhydroxyethylaspanamidephenol, or polyethyleneoxidepolylysine substituted with palmitoyl residues. Furthermore, the compounds of the present invention may be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates and cross-linked or amphipathic block copolymers of hydro gels.

If desired, the pharmaceutical composition to be administered may also contain minor amounts of non-toxic auxiliary substances such as wetting or emulsifying agents, pH buffering agents, and other substances such as for example, sodium acetate, and triethanolamine oleate.

The dosage regimen utilizing the aptamers is selected in accordance with a variety of factors including type, species, age, weight, sex and medical condition of the patient; the severity of the condition to be treated; the route of administration; the renal and hepatic function of the patient; and the particular aptamer or salt thereof employed. An ordinarily skilled physician or veterinarian can readily determine and prescribe the effective amount of the drug required to prevent, counter or arrest the progress of the condition.

Oral dosages of the present invention, when used for the indicated effects, will range between about 0.05 to 7500 mg/day orally. The compositions are preferably provided in the form of scored tablets containing 0.5,1.0,2.5, 5.0,10.0,15.0, 25.0, 50.0,100.0,250.0, 500.0 and 1000.0 mg of active ingredient. Infused dosages, intranasal dosages and transdermal dosages will range between 0.05 to 7500 mg/day. Subcutaneous, intravenous and intraperitoneal dosages will range between 0.05 to 3800 mg/day.

Effective plasma levels of the compounds of the present invention range from 0.002 mg/mL to 50 mg/mL.

Compounds of the present invention may be administered in a single daily dose, or the total daily dosage may be administered in divided doses of two, three or four times daily.

### Modulation of pharmacokinetics and biodistribution of aptamer therapeutics

It is important that the pharmacokinetic properties for all oligonucleotide-based therapeutics, including aptamers, be tailored to match the desired pharmaceutical application. While aptamers directed against extracellular targets do not suffer from difficulties associated with intracellular delivery (as is the case with antisense and RNAi-based therapeutics), such aptamers must still be able to be distributed to target organs and tissues, and remain in the body (unmodified) for a period of time consistent with the desired dosing regimen.

Thus, the present invention provides materials and methods to affect the pharmacokinetics of aptamer compositions, and, in particular, the ability to tune aptamer pharmacokinetics. The tunability of *(i.e.,* the ability to modulate) aptamer pharmacokinetics is achieved through conjugation of modifying moieties (*e.g*., PEG polymers) to the aptamer and/or the incorporation of modified nucleotides (*e.g*., 2'-fluoro or 2'-O-methyl) to alter the chemical composition of the nucleic acid. The ability to tune aptamer pharmacokinetics is used in the improvement of existing therapeutic applications, or alternatively, in the development of new therapeutic applications. For example, in some therapeutic applications, *e.g*., in anti-neoplastic or acute care settings where rapid drug clearance or turn-off may be desired, it is desirable to decrease the residence times of aptamers in the circulation. Alternatively, in other therapeutic applications, *e.g*., maintenance therapies where systemic circulation of a therapeutic is desired, it may be desirable to increase the residence times of aptamers in circulation.

In addition, the tunability of aptamer pharmacokinetics is used to modify the biodistribution of an aptamer therapeutic in a subject. For example, in some therapeutic applications, it may be desirable to alter the biodistribution of an aptamer therapeutic in an effort to target a particular type of tissue or a specific organ (or set of organs). In these applications, the aptamer therapeutic preferentially accumulates in a specific tissue or organ(s). In other therapeutic applications, it may be desirable to target tissues displaying a cellular marker or a symptom associated with a given disease, cellular injury or other abnormal pathology, such that the aptamer therapeutic preferentially accumulates in the affected tissue. For example, as described in copending provisional application United States Serial No. 60/550,790, filed on March 5, 2004, and entitled "Controlled Modulation of the Pharmacokinetics and Biodistribution of Aptamer Therapeutics", and in the non-provisional application United States Serial No. 11/075,648, filed on March 7, 2005, also entitled "Controlled Modulation of the Pharmacokinetics and Biodistribution of Aptamer Therapeutics", PEGylation of an aptamer therapeutic (*e.g*., PEGylation with a 20 kDa PEG polymer) is used to target inflamed tissues, such that the PEGylated aptamer therapeutic preferentially accumulates in inflamed tissue.

To determine the pharmacokinetic and biodistribution profiles of aptamer therapeutics (*e.g*., aptamer conjugates or aptamers having altered chemistries, such as modified nucleotides) a variety of parameters are monitored. Such parameters include, for example, the half-life (t1/2), the plasma clearance (C1), the volume of distribution (Vss), the area under the concentration-time curve (AUC), maximum observed serum or plasma concentration (Cmax), and the mean residence time (MRT) of an aptamer composition. As used herein, the term "AUC" refers to the area under the plot of the plasma concentration of an aptamer therapeutic versus the time after aptamer administration. The AUC value is used to estimate the bioavailability (*i.e*., the percentage of administered aptamer therapeutic in the circulation after aptamer administration) and/or total clearance (C1) *(i.e.,* the rate at which the aptamer therapeutic is removed from circulation) of a given aptamer therapeutic. The volume of distribution relates the plasma concentration of an aptamer therapeutic to the amount of aptamer present in the body. The larger the Vss, the more an aptamer is found outside of the plasma (*i.e*., the more extravasation).

The present invention provides materials and methods to modulate, in a controlled manner, the pharmacokinetics and biodistribution of stabilized aptamer compositions *in vivo* by conjugating an aptamer to a modulating moiety such as a small molecule, peptide, or polymer terminal group, or by incorporating modified nucleotides into an aptamer. As described herein, conjugation of a modifying moiety and/or altering nucleotide(s) chemical composition alters fundamental aspects of aptamer residence time in circulation and distribution to tissues.

In addition to clearance by nucleases, oligonucleotide therapeutics are subject to elimination via renal filtration. As such, a nuclease-resistant oligonucleotide administered intravenously typically exhibits an *in vivo* half-life of <10 min, unless filtration can be blocked. This can be accomplished by either facilitating rapid distribution out of the blood stream into tissues or by increasing the apparent molecular weight of the oligonucleotide above the effective size cut-off for the glomerulus. Conjugation of small therapeutics to a PEG polymer (PEGylation), described below, can dramatically lengthen residence times of aptamers in circulation, thereby decreasing dosing frequency and enhancing effectiveness against vascular targets.

Aptamers can be conjugated to a variety of modifying moieties, such as high molecular weight polymers, e.g., PEG; peptides, e.g., Tat (a 13-amino acid fragment of the HIV Tat protein (Vives, et al., (1997), J. Biol. Chem. 272(25): 16010-7)), Ant (a 16-amino acid sequence derived from the third helix of the Drosophila antennapedia homeotic protein (Pietersz, et al., (2001), Vaccine 19(11-12): 1397-405)) and Arg7 (a short, positively charged cell-permeating peptides composed of polyarginine (Arg7) (Rothbard, et al., (2000), Nat. Med. 6(11): 1253-7; Rothbard, J et al., (2002), J. Med. Chem. 45(17): 3612-8)); and small molecules*, e.g.,* lipophilic compounds such as cholesterol. Among the various conjugates described herein, *in vivo* properties of aptamers are altered most profoundly by complexation with PEG groups. For example, complexation of a mixed 2'F and 2'-OMe modified aptamer therapeutic with a 20 kDa PEG polymer hinders renal filtration and promotes aptamer distribution to both healthy and inflamed tissues. Furthermore, the 20 kDa PEG polymer-aptamer conjugate proves nearly as effective as a 40 kDa PEG polymer in preventing renal filtration of aptamers. While one effect of PEGylation is on aptamer clearance, the prolonged systemic exposure afforded by presence of the 20 kDa moiety also facilitates distribution of aptamer to tissues, particularly those of highly perfused organs and those at the site of inflammation. The aptamer-20 kDa PEG polymer conjugate directs aptamer distribution to the site of inflammation, such that the PEGylated aptamer preferentially accumulates in inflamed tissue. In some instances, the 20 kDa PEGylated aptamer conjugate is able to access the interior of cells, such as, for example, kidney cells.

Modified nucleotides can also be used to modulate the plasma clearance of aptamers. For example, an unconjugated aptamer which incorporates both 2'-F and 2'-OMe stabilizing chemistries, which is typical of current generation aptamers as it exhibits a high degree of nuclease stability *in vitro* and *in vivo,* displays rapid loss from plasma *(i.e.,* rapid plasma clearance) and a rapid distribution into tissues, primarily into the kidney, when compared to unmodified aptamer.

### PEG-Derivatized Nucleic Acids

As described above, derivatization of nucleic acids with high molecular weight non-immunogenic polymers has the potential to alter the pharmacokinetic and pharmacodynamic properties of nucleic acids making them more effective therapeutic agents. Favorable changes in activity can include increased resistance to degradation by nucleases, decreased filtration through the kidneys, decreased exposure to the immune system, and altered distribution of the therapeutic through the body.

The aptamer compositions of the invention may be derivatized with polyalkylene glycol ("PAG") moieties. Examples of PAG-derivatized nucleic acids are found in United States Patent Application Ser. No. 10/718,833, filed on November 21, 2003, . Typical polymers used in the invention include polyethylene glycol ("PEG"), also known as polyethylene oxide ("PEO") and polypropylene glycol (including poly isopropylene glycol). Additionally, random or block copolymers of different alkylene oxides (*e.g*., ethylene oxide and propylene oxide) can be used in many applications. In its most common form, a polyalkylene glycol, such as PEG, is a linear polymer terminated at each end with hydroxyl groups: HO-CH2CH2O-(CH2CH2O) n-CH2CH2-OH. This polymer, alpha-, omega-dihydroxylpolyethylene glycol, can also be represented as HO-PEG-OH, where it is understood that the —PEG- symbol represents the following structural unit: -CH2CH2O-(CH2CH2O) n-CH2CH2- where n typically ranges from about 4 to about 10,000.

As shown, the PEG molecule is di-functional and is sometimes referred to as "PEG diol." The terminal portions of the PEG molecule are relatively non-reactive hydroxyl moieties, the -OH groups, that can be activated, or converted to functional moieties, for attachment of the PEG to other compounds at reactive sites on the compound. Such activated PEG diols are referred to herein as bi-activated PEGs. For example, the terminal moieties of PEG diol have been functionalized as active carbonate ester for selective reaction with amino moieties by substitution of the relatively non-reactive hydroxyl moieties, -OH, with succinimidyl active ester moieties from N-hydroxy succinimide.

In many applications, it is desirable to cap the PEG molecule on one end with an essentially non-reactive moiety so that the PEG molecule is mono-functional (or mono-activated). In the case of protein therapeutics which generally display multiple reaction sites for activated PEGs, bi-functional activated PEGs lead to extensive cross-linking, yielding poorly functional aggregates. To generate mono-activated PEGs, one hydroxyl moiety on the terminus of the PEG diol molecule typically is substituted with non-reactive methoxy end moiety, -OCH3. The other, un-capped terminus of the PEG molecule typically is converted to a reactive end moiety that can be activated for attachment at a reactive site on a surface or a molecule such as a protein.

PAGs are polymers which typically have the properties of solubility in water and in many organic solvents, lack of toxicity, and lack of immunogenicity. One use of PAGs is to covalently attach the polymer to insoluble molecules to make the resulting PAG-molecule "conjugate" soluble. For example, it has been shown that the water-insoluble drug paclitaxel, when coupled to PEG, becomes water-soluble. Greenwald, et al., J. Org. Chem., 60:331-336 (1995). PAG conjugates are often used not only to enhance solubility and stability but also to prolong the blood circulation half-life of molecules.

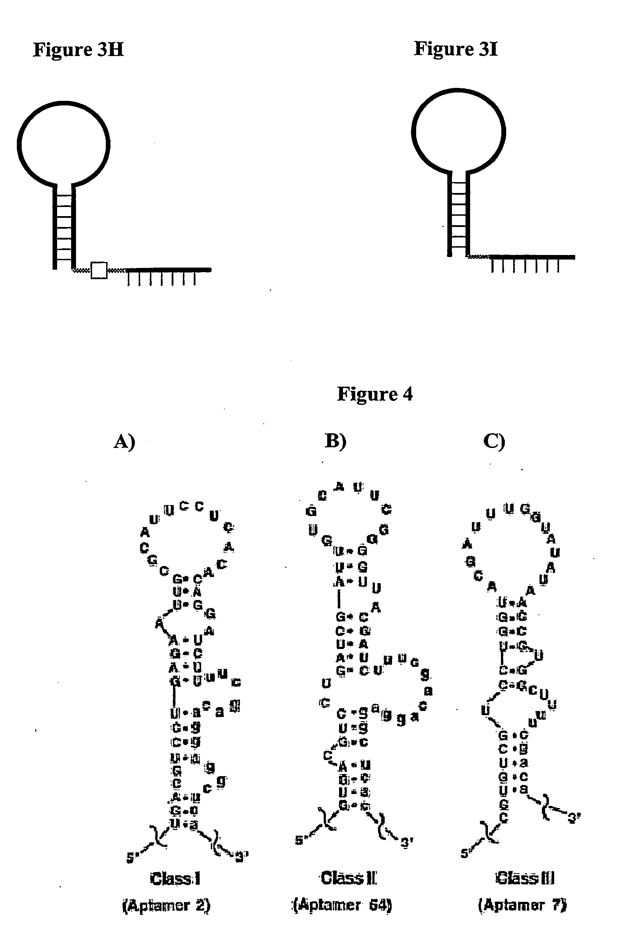

Polyalkylated compounds of the invention are typically between 5 and 80 kDa in size however any size can be used, the choice dependent on the aptamer and application. Other PAG compounds of the invention are between 10 and 80 kDa in size. Still other PAG compounds of the invention are between 10 and 60 kDa in size. For example, a PAG polymer may be at least 10, 20, 30, 40, 50, 60, or 80 kDa in size. Such polymers can be linear or branched. In some embodiments the polymers are PEG. In some embodiment the polymers are branched PEG. In still other embodiments the polymers are 40kDa branched PEG as depicted in Figure 4. In some embodiments the 40 kDa branched PEG is attached to the 5' end of the aptamer as depicted in Figure 5.

In contrast to biologically-expressed protein therapeutics, nucleic acid therapeutics are typically chemically synthesized from activated monomer nucleotides. PEG-nucleic acid conjugates may be prepared by incorporating the PEG using the same iterative monomer synthesis. For example, PEGs activated by conversion to a phosphoramidite form can be incorporated into solid-phase oligonucleotide synthesis. Alternatively, oligonucleotide synthesis can be completed with site-specific incorporation of a reactive PEG attachment site. Most commonly this has been accomplished by addition of a free primary amine at the 5'-terminus (incorporated using a modifier phosphoramidite in the last coupling step of solid phase synthesis). Using this approach, a reactive PEG (e.g., one which is activated so that it will react and form a bond with an amine) is combined with the purified oligonucleotide and the coupling reaction is carried out in solution.

The ability of PEG conjugation to alter the biodistribution of a therapeutic is related to a number of factors including the apparent size (e.g., as measured in terms of hydrodynamic radius) of the conjugate. Larger conjugates (>10 kDa) are known to more effectively block filtration via the kidney and to consequently increase the serum half-life of small macromolecules (e.g., peptides, antisense oligonucleotides). The ability of PEG conjugates to block filtration has been shown to increase with PEG size up to approximately 50 kDa (further increases have minimal beneficial effect as half life becomes defined by macrophage-mediated metabolism rather than elimination via the kidneys).

Production of high molecular weight PEGs (>10 kDa) can be difficult, inefficient, and expensive. As a route towards the synthesis of high molecular weight PEG-nucleic acid conjugates, previous work has been focused towards the generation of higher molecular weight activated PEGs. One method for generating such molecules involves the formation of a branched activated PEG in which two or more PEGs are attached to a central core carrying the activated group. The terminal portions of these higher molecular weight PEG molecules, i.e., the relatively non-reactive hydroxyl (-OH) moieties, can be activated, or converted to functional moieties, for attachment of one or more of the PEGs to other compounds at reactive sites on the compound. Branched activated PEGs will have more than two termini, and in cases where two or more termini have been activated, such activated higher molecular weight PEG molecules are referred to herein as, multi-activated PEGs. In some cases, not all termini in a branch PEG molecule are activated. In cases where any two termini of a branch PEG molecule are activated, such PEG molecules are referred to as bi-activated PEGs. In some cases where only one terminus in a branch PEG molecule is activated, such PEG molecules are referred to as mono-activated. As an example of this approach, activated PEG prepared by the attachment of two monomethoxy PEGs to a lysine core which is subsequently activated for reaction has been described (Harris et al., Nature, vol.2: 214-221, 2003).

The present invention provides another cost effective route to the synthesis of high molecular weight PEG-nucleic acid (preferably, aptamer) conjugates including multiply PEGylated nucleic acids. The present invention also encompasses PEG-linked multimeric oligonucleotides, e.g., dimerized aptamers. The present invention also relates to high molecular weight compositions where a PEG stabilizing moiety is a linker which separates different portions of an aptamer, e.g., the PEG is conjugated within a single aptamer sequence, such that the linear arrangement of the high molecular weight aptamer composition is, e.g., nucleic acid - PEG - nucleic acid (- PEG - nucleic acid)n where n is greater than or equal to 1.

High molecular weight compositions of the invention include those having a molecular weight of at least 10 kDa. Compositions typically have a molecular weight between 10 and 80 kDa in size. High molecular weight compositions of the invention are at least 10, 20, 30, 40, 50, 60, or 80 kDa in size.

A stabilizing moiety is a molecule, or portion of a molecule, which improves pharmacokinetic and pharmacodynamic properties of the high molecular weight aptamer compositions of the invention. In some cases, a stabilizing moiety is a molecule or portion of a molecule which brings two or more aptamers, or aptamer domains, into proximity, or provides decreased overall rotational freedom of the high molecular weight aptamer compositions of the invention. A stabilizing moiety can be a polyalkylene glycol, such a polyethylene glycol, which can be linear or branched, a homopolymer or a heteropolymer. Other stabilizing moieties include polymers such as peptide nucleic acids (PNA). Oligonucleotides can also be stabilizing moieties; such oligonucleotides can include modified nucleotides, and/or modified linkages, such as phosphorothioates. A stabilizing moiety can be an integral part of an aptamer composition, *i.e*., it is covalently bonded to the aptamer.

Compositions of the invention include high molecular weight aptamer compositions in which two or more nucleic acid moieties are covalently conjugated to at least one polyalkylene glycol moiety. The polyalkylene glycol moieties serve as stabilizing moieties. In compositions where a polyalkylene glycol moiety is covalently bound at either end to an aptamer, such that the polyalkylene glycol joins the nucleic acid moieties together in one molecule, the polyalkylene glycol is said to be a linking moiety. In such compositions, the primary structure of the covalent molecule includes the linear arrangement nucleic acid-PAG-nucleic acid. One example is a composition having the primary structure nucleic acid-PEG-nucleic acid. Another example is a linear arrangement of: nucleic acid ― PEG - nucleic acid - PEG - nucleic acid.

To produce the nucleic acid—PEG—nucleic acid conjugate, the nucleic acid is originally synthesized such that it bears a single reactive site (*e.g*., it is mono-activated). In a preferred embodiment, this reactive site is an amino group introduced at the 5'-terminus by addition of a modifier phosphoramidite as the last step in solid phase synthesis of the oligonucleotide. Following deprotection and purification of the modified oligonucleotide, it is reconstituted at high concentration in a solution that minimizes spontaneous hydrolysis of the activated PEG. In a preferred embodiment, the concentration of oligonucleotide is 1 mM and the reconstituted solution contains 200 mM NaHCO3-buffer, pH 8.3. Synthesis of the conjugate is initiated by slow, step-wise addition of highly purified bi-functional PEG. In a preferred embodiment, the PEG diol is activated at both ends (bi-activated) by derivatization with succinimidyl propionate. Following reaction, the PEG-nucleic acid conjugate is purified by gel electrophoresis or liquid chromatography to separate fully-, partially-, and un-conjugated species. Multiple PAG molecules concatenated (*e.g*., as random or block copolymers) or smaller PAG chains can be linked to achieve various lengths (or molecular weights). Non-PAG linkers can be used between PAG chains of varying lengths.

The 2'-O-methyl, 2'-fluoro and other modified nucleotide modifications stabilize the aptamer against nucleases and increase its half life *in vivo*. The 3'-3'-dT cap also increases exonuclease resistance. See, *e.g*., U.S. Patents 5,674,685; 5,668,264; 6,207,816; and 6,229,002.

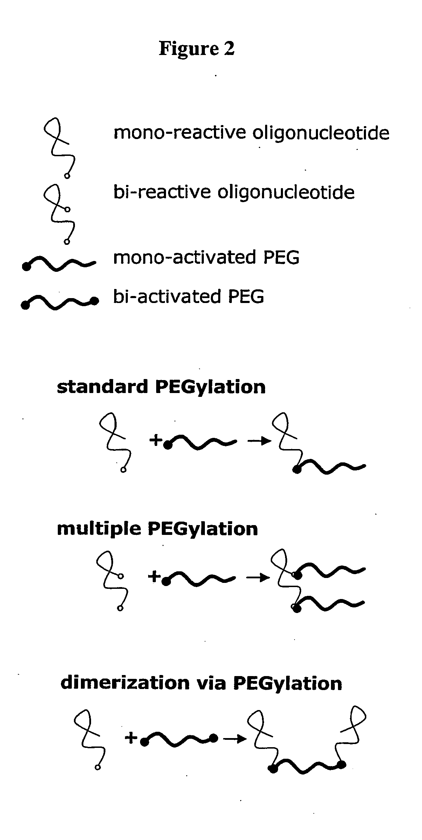

High molecular weight PAG-nucleic acid-PAG conjugates can be prepared by reaction of a mono-functional activated PEG with a nucleic acid containing more than one reactive site. In one embodiment, the nucleic acid is bi-reactive, or bi-activated, and contains two reactive sites: a 5'-amino group and a 3'-amino group introduced into the oligonucleotide through conventional phosphoramidite synthesis, for example: 3'-5'-di-PEGylation as illustrated in Figure 2. In alternative embodiments, reactive sites can be introduced at internal positions, using for example, the 5-position of pyrimidines, the 8-position of purines, or the 2'-position of ribose as sites for attachment of primary amines. In such embodiments, the nucleic acid can have several activated or reactive sites and is said to be multiply activated. Following synthesis and purification, the modified oligonucleotide is combined with the mono-activated PEG under conditions that promote selective reaction with the oligonucleotide reactive sites while minimizing spontaneous hydrolysis. In the preferred embodiment, monomethoxy-PEG is activated with succinimidyl propionate and the coupled reaction is carried out at pH 8.3. To drive synthesis of the bi-substituted PEG, stoichiometric excess PEG is provided relative to the oligonuleotide. Following reaction, the PEG-nucleic acid conjugate is purified by gel electrophoresis or liquid chromatography to separate fully-, partially-, and un-conjugated species. Figure 2 illustrates two strategies for synthesizing PEGylated nucleic acid aptamers.

The linking domains can also have one or more polyalkylene glycol moieties attached thereto. Such PAGs can be of varying lengths and may be used in appropriate combinations to achieve the desired molecular weight of the composition.

### Intracellular Delivery of Therapeutic Agents

The present invention provides materials and methods to mediate the intracellular delivery of therapeutic agents, such as therapeutic oligonucleotides. In one embodiment, the invention provides a bi-functional therapeutic oligonucleotide that includes a delivery aptamer, referred to herein as "Domain I", linked to a therapeutic oligonucleotide, referred to herein as "Domain II". The delivery aptamer and the therapeutic oligonucleotide are linked, for example, covalently or functionally through DNA/RNA or DNA/DNA duplex formation. In one embodiment of the invention, the bifunctional therapeutic oligonucleotide may be partly or wholly comprised of 2'-modified RNA or DNA such as 2'F, 2'OH, 2'OMe, 2'allyl, 2'MOE substituted nucleotides, and may contain PEG-spacers and abasic residues.

The bi-functional therapeutic oligonucleotide of this invention uses nucleic acid aptamers (that have been developed against extracellular targets) as a delivery vehicle, for other therapeutic oligonucleotide-derived drugs, to the intracellular environment. Inside the cell, such oligonucleotide-derived drugs can then target either messenger RNA (mRNA) or its protein product.

The internalization of nucleic acid constructs having a delivery aptamer has been shown, *e.g*., by Farokhzad et al., Cancer Res. vol. 64:7668-72 (2004).
In this study, nanoparticle-aptamer bioconjugate having RNA aptamers that bind to prostate specific membrane antigen (PSMA) were generated. These nanoparticle aptamer bioconjugates were created using poly(lactic-acid)-block PEG copolymers having a terminal carboxylic acid functional group (PLA-PEG-COOH) to encapsulate rhodamine-labeled dextran (as a model drug). These nanoparticles were then conjugated to the A10 PSMA aptamer (described below). As shown by Farokhzad *et al.,* these nanoparticle-aptamer bioconjugates were efficiently taken up by prostate LNCaP epithelial cells, which express the PSMA protein. Thus, these conjugates provided selective and efficient drug delivery to targeted cells.

The therapeutic oligonucleotide-derived drugs of the invention are used to treat disease in several ways. Short, single-stranded antisense RNAs inhibit gene expression by binding complimentary mRNAs and blocking translation or by causing the degradation of the mRNA transcript by activating RNaseH. Short double-stranded RNAs (siRNAs) activate the RNA interference (RNAi) pathway leading to the degradation of complimentary mRNA. Ribozymes inhibit gene expression by cleaving mRNA molecules rendering them inactive. Aptamers and decoys targeted to proteins bind and inactivate those proteins in a manner analogous to small molecule drugs. In one embodiment, the bi-functional therapeutic oligonucleotides of the invention are used in the treatment of cancer as an anti-proliferative agent.

In contrast to the therapeutic oligonucleotides of the invention, earlier work has described individual molecules as therapeutic molecules, e.g., either aptamers (as therapeutic or detection molecules), anti-sense sequences as therapeutic molecules, siRNA sequences as therapeutic molecules, ribozymes as therapeutic molecules, decoys as therapeutic molecules, or CpG oligonucleotides as therapeutic molecules.

A limitation of the use of these individual oligonucleotide molecules for therapeutic purposes has been the difficulty of delivering these macromolecules into the intracellular environment. This has been especially problematic for larger RNA, DNA or modified oligonucleotide molecules such as siRNAs, antisense, ribozymes, decoys and aptamers targeted to intracellular proteins. Methods of intracellular delivery of these larger therapeutic oligonucleotides have included passive uptake, liposome mediated transfection, conjugation to peptides (*e.g*., antenopedia, polyArg) and conjugation to small molecules (*e.g.,* valine, folate, glcNac) that are actively or passively transported into cells.

With the exception of the CpG oligonucleotides described herein, the therapeutic oligonucleotides of the invention are not specifically modified with additional nucleic acid sequences that are able to secure and drive cellular uptake. Rather, the bifunctional, therapeutic oligonucleotides of the invention include a nucleic delivery sequence that is able to drive uptake and cellular internalization of a second oligonucleotide.

The bi-functional therapeutic oligonucleotides of the invention target a therapeutic to specific cell types. For example, PSMA aptamers target the therapeutic oligonucleotide to hormone-independent prostate tumor cells, and thereby deliver siRNAs, antisense, ribozymes, decoys or therapeutic aptamers that target anti-apoptotic proteins (*e.g*., BCL2, MDM-2) or proteins which drive cellular proliferation (*e.g*., oncogenes, PCNA, kinases, transcription factors).

As both the delivery aptamer and the therapeutic oligonucleotide are comprised of nucleic acid sequences, the bifunctional therapeutic oligonucleotides are generated through similar synthetic methods. In addition, the delivery aptamer and the therapeutic oligonucleotide can each be generated separately and then chemically joined in a final synthetic step.

### Domain I

In one embodiment of the invention, the delivery aptamer binds to and is internalized via its interaction with a cell surface protein such as a tumor antigen. In another embodiment, the delivery aptamer is an aptamer that binds to the tumor antigen PSMA such as the aptamers described by Coffey *et al.,* and the PSMA binding aptamers described herein, or such as the aptamers that bind to Her2/Neu, EGFRI-III, MUC-1, CD4, cMET, HERIII, CEA, or CD22 and CD33 tumor antigens.

In one embodiment of the invention, the delivery aptamer binds to and is internalized via its interaction with a cell surface protein such as a growth factor receptor. In another embodiment, the delivery aptamer is an aptamer that binds to the VEGF receptors VEGFRI and VEGFRII such as the aptamers described herein.

In one embodiment of the invention, the delivery aptamer binds to and is internalized via its interaction with a complex formed from a growth factor bound to its receptor. Many growth factor-receptor complexes are internalized as a means of receptor down regulation (*e.g*., EGF-EGFR, VEGF-VEGFR, HCG-cMET). Hence, aptamer-mediated delivery via binding to the activated receptor complex provides a novel means of specifically targeting cells that are activated under a pathological condition.

In one embodiment of the invention, the delivery aptamer binds to and is internalized via its interaction with a cell surface protein such as a cytokine or chemokine receptor.

In one embodiment of the invention, the delivery aptamer binds to and is internalized via its interaction with a cell surface protein such as G-protein coupled receptor. In another embodiment, the delivery aptamer is an aptamer that binds to the neurotensin NTS-1 protein.

In one embodiment of the invention, the delivery aptamer binds to and is internalized via its interaction with a cytokine-cytokine receptor complex. Many cytokine mediated GPCRs are internalized upon ligand binding. Hence, aptamer-mediated delivery via binding to the activated GPCR complex provides a novel means of specifically targeting GPCR with aptamers and also specifically targeting cells that are activated under a pathological condition.

In one embodiment of the invention, the delivery aptamer binds to and is internalized via its interaction with a viral coat protein. In another embodiment, the delivery aptamer is an aptamer that binds to the gp120 coat protein of HIV, or to the gp41 fusion peptide of HIV such as the aptamers described herein.

In one embodiment of the invention, the delivery-aptamer (domain I) is a CpG oligonucleotide.

Other targets that the delivery aptamer binds to and is internalized via includes, for example, CD6, CD19, CD22, CD23, CD33, CD44v6, CD56, TENB2, HER2/neu (also referred to as erb2), EGF receptor vIII, Cripto-1, and Alpha(v)beta(3/5).

Examples of aptamers that bind to cell surface antigens that internalize are shown below in Table 1.

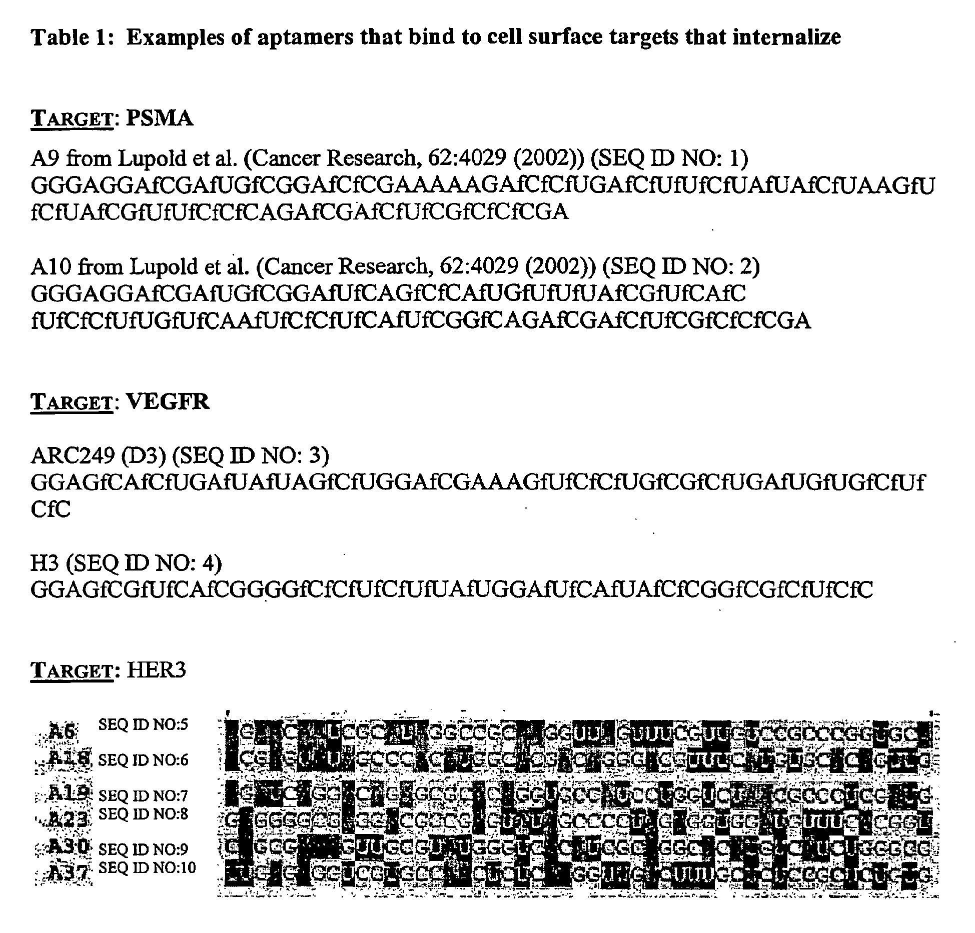

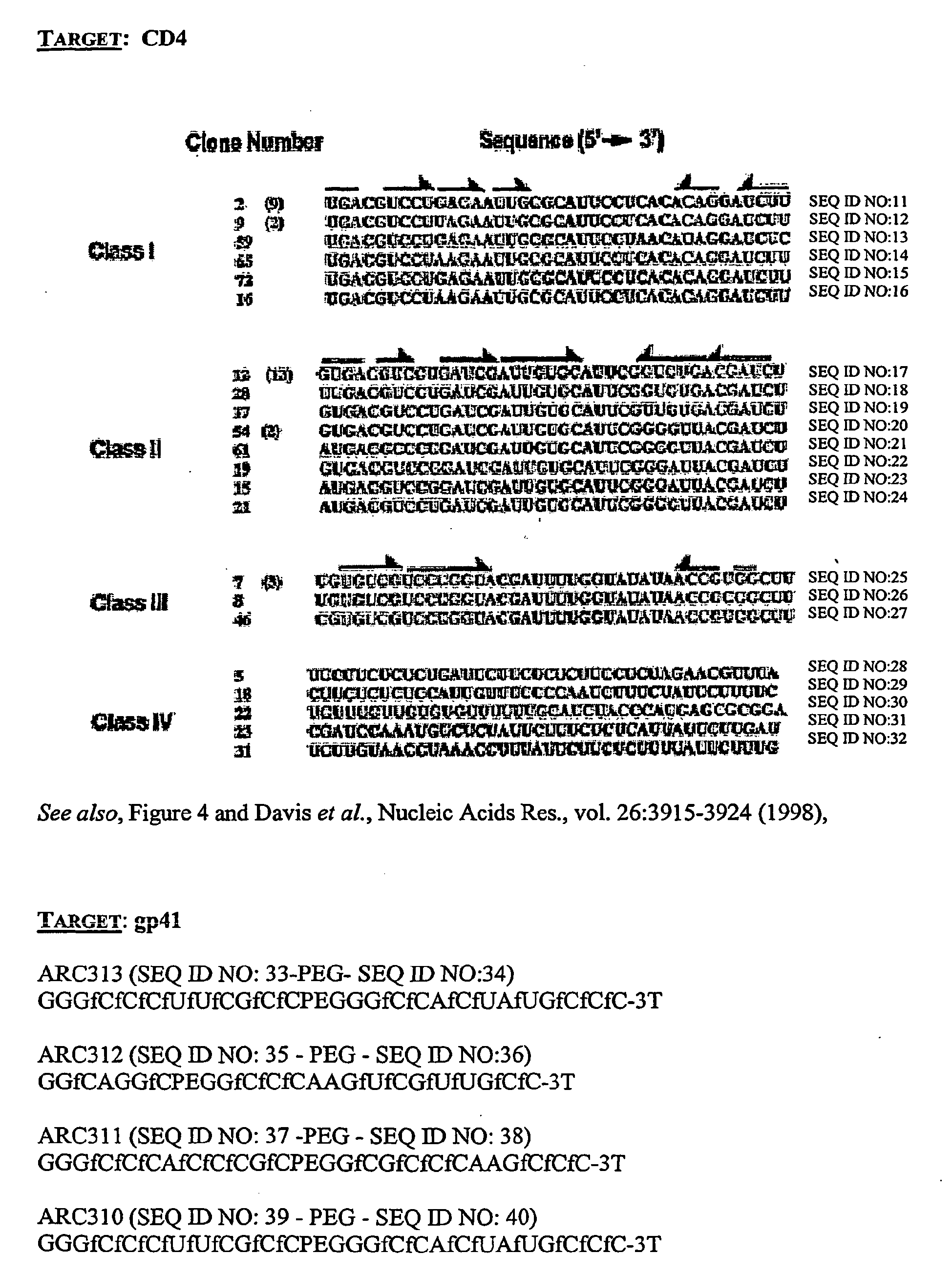

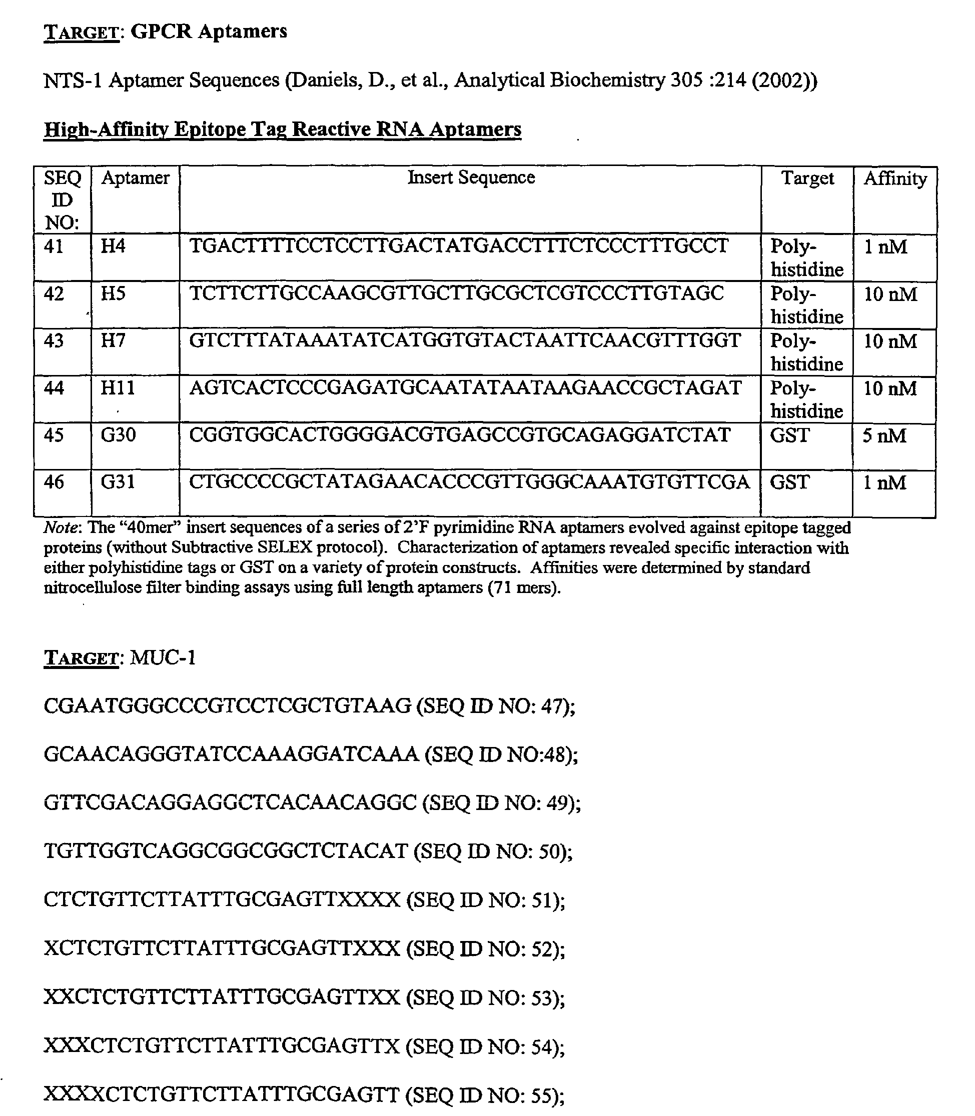

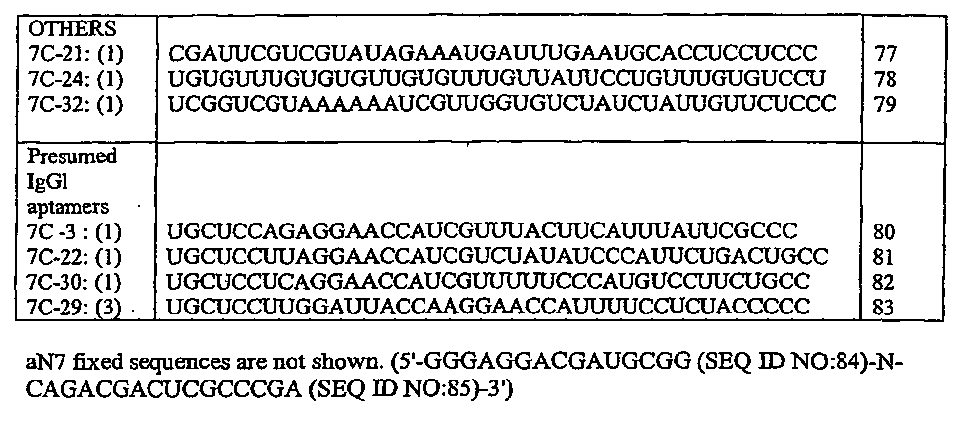

### Linking Moiety

In one embodiment of the invention, there is a variable linker region (1-30 residues in length) that attaches the delivery aptamer and the therapeutic oligonucleotide. In one embodiment, the linker includes DNA, RNA, or any combination of 2'-modified nucleotide residues. For example, the linker is simply a phosphodiester linkage. Alternatively, the nucleic acid linker has a length in the range of less than 10 nucleotides, less than 20, or less than 50 nucleotides. Suitable linkers can include modified nucleotides or modified backbone. The linker region is double or single stranded nucleic acid and is designed to release the therapeutic oligonucleotide by endonucleolytic cleavage, subsequent to internalization of the bifunctional aptamer or to be acid labile such that it is released upon internalization into endosomes. The linker is a random sequence. In one embodiment, the linker is a DNA/RNA hybrid or other target subject to RNAse H cleavage. In another embodiment, the linker is peptidic in nature, or includes a single or PNA residues. In another embodiment, the linker is formed of a disulfide bond that is subsequently cleaved upon exposure to intracellular thiol agents such as glutathione. In another embodiment, the linker is a polyethylene glycol moiety.

### Domain II

This invention describes the generation and use of a bifunctional therapeutic oligonucleotide, where the delivery aptamer (Domain I) functions to deliver or transport a therapeutic oligonucleotide, Domain II, to the interior of the target cell.

**SiRNA Molecules**: In the invention, the therapeutic oligonucleotide is an siRNA oligonucleotide which is linked to the delivery aptamer. As used herein, the term "siRNA oligonucleotide" includes a coding strand and a noncoding strand. In one embodiment of the invention, the coding strand of the siRNA oligonucleotide is covalently attached to the delivery aptamer through a 5'-3' phosphodiester linkage, and the noncoding strand of the siRNA is bound through Watson-Crick base-paring to the coding strand. In another embodiment of the invention, the coding strand of the siRNA oligonucleotide is covalently attached to the delivery aptamer through a 5'-3' phosphodiester linkage to the 5'-end of the delivery aptamer, and the noncoding strand of the siRNA is bound through Watson-Crick base-paring to the coding strand. In another embodiment of the invention, the coding strand of the siRNA oligonucleotide is covalently attached to the delivery aptamer through a 5'-3' phosphodiester linkage to the 3'-end of the delivery aptamer, and the noncoding strand of the siRNA is bound through Watson-Crick base-paring to the coding strand.

In another embodiment of the invention, the coding and noncoding strands of the siRNA oligonucleotide are formed from a contiguous sequence which is covalently attached to the delivery aptamer through a 5'-3' phosphodiester linkage to either the 5'- or 3'-end of the delivery aptamer.

In one embodiment of the invention, the siRNA is specific for a target including, by way of non-limiting example, proliferating cell nuclear antigen (PCNA), Androgen Receptor, BCL2, NFkB, VEGFR1, VEGFR2, VEGFR3, HER2/neu, c-Myc, REL-A, PTP1B, BACE, CHK1, PKC-alpha, EGFR, CHK-1, ERG2, Cyclin D1, CCND1 P, KCa, RAF1, MAPK1, MAPK8, MYB, KRAS2, KDR, IKKg, hTR, HRAS, FOS, GAB2, FLT1, EZH2 or CDK2.

siRNA that target Androgen Receptor include, for example, AAGAAGGCCAGUUGUAUGGAC (SEQ ID NO:86) and AAGACGCUUCUACCAGCUCAC (SEQ ID NO:87) *(See e.g.,* Liao et al., Mol. Cancer Ther. vol. 4(4): 505-15 (2005)).

siRNA molecules that target HER2 include, e.g., siRNA having the following sequences: GGUGCUUGGAUCUGGCGCUTT (SEQ ID NO:88); AGCGCCAGAUCCAAGCACCTT (SEQ ID NO:89); UCGCGGUCUAGGUUCGUGGTT (SEQ ID NO: 90); CCACGAACCUAGACCGCGATT (SEQ ID NO:91); UGGGGUCGUCAAAGACGUUTT (SEQ ID NO:92); AACGUCUUUGACGACCCCATT (SEQ ID NO:93); UUGCAGAAACUGCUGGGGUTT (SEQ ID NO:94); ACCCCAGCAGUUUCUGCAATT (SEQ ID NO:95); B GGuGcuuGGAucuGGcGcuTT B (SEQ ID NO:96); AGcGccAGAuccAAGcAccTsT (SEQ ID NO:97); B ucGcGGucuAGGuucGuGGTT B (SEQ ID NO:98); ccAcGAAccuAGAccGcGATsT (SEQ ID NO:99); B uGGGGucGucAAAGAcGuuTT B (SEQ ID NO:100); AAcGucuuuGAcGAccccATsT (SEQ ID NO:101); B uuGcAGAAAcuGcuGGGGuTT B (SEQ ID NO:102); AccccAGcAGuuucuGcAATsT (SEQ ID NO:103), where the nucleotides presented in uppercase are ribonucleotides, lower case nucleotides "u" and lower case "c" signify 2'-deoxy-2'-fluoro U and 2'-deoxy-2'-fluoro C residues, T is thymidine, B is an inverted deoxy abasic residue, and s is a phosphorthioate linkage. *(See e.g.,* PCT Publication No. WO 03/074654, published September 12, 2003).

siRNA molecules that target EGFR include, for example, the following sequences: CCAAGUCCUACAGACUCCATT (SEQ ID NO:104); UGGAGUCUGUAGGACUUGGTT (SEQ ID NO:105); B ccAAGuccuAcAGAcuccATT B (SEQ ID NO:106) and uGGAGucuGuAGGAcuuGGTsT (SEQ ID NO:107), where the nucleotides presented in uppercase are ribonucleotides, lower case nucleotides "u" and lower case "c" signify 2'-deoxy-2'-fluoro U and 2'-deoxy-2'-fluoro C residues, T is thymidine, B is an inverted deoxy abasic residue, and s is a phosphorthioate linkage *(See e.g.,* PCT Publication No. WO 03/074654)

Suitable siRNA molecules that target PKC-alpha include, e.g., AAAGGCUGAGGUUGCUGAUTT (SEQ ID NO: 108); AUCAGCAACCUCAGCCUUUTT (SEQ ID NO:109); AAACAACCUUCCAACAACCTT (SEQ ID NO:110); GGUUGUUGGAAGGUUGUUUTT (SEQ ID NO: 111); GGAUGUGGUGAUUCAGGAUTT (SEQ ID NO:112); AUCCUGAAUCACCACAUCCTT (SEQ ID NO:113); AAGGACUGAUGACCAAACATT (SEQ ID NO: 114); and UGUUUGGUCAUCAGUCCUUTT (SEQ ID NO: 115) *(See e.g.,* PCT Publication No. WO 03/074654).

siRNA molecules that target PCNA include, for example, the following molecules: AACAGCAUCUCCAAUAUGGTT (SEQ ID NO:116); AGUGUCCCAUAUCCGCAAUTT (SEQ ID NO:117); B uucucucAccucAccGAAATT B (SEQ ID NO:118) and uuucGGuGAGGuGAGAGAATsT (SEQ ID NO: 119), where the nucleotides presented in uppercase are ribonucleotides, lower case nucleotides "u" and lower case "c" signify 2'-deoxy-2'-fluoro U and 2'-deoxy-2'-fluoro C residues, T is thymidine, B is an inverted deoxy abasic residue, and s is a phosphorthioate linkage *(See e.g.,* PCT Publication No. WO 03/074654).

In one embodiment, the siRNA is specific for PCNA and has the following construct, referred to herein as "Construct A": RPI#31035/31111, which includes ribonucleotides and 3' terminal dithymidine caps, wherein 31035 has the nucleic acid sequence AAAGccAcuccAcucucuuTT (SEQ ID NO:120) and 31111 has the nucleic acid sequence AAGAGAGuGGAGuGGcuuuTT (SEQ ID NO:121), where the nucleotides presented in uppercase are ribonucleotides and lower case nucleotides "u" and lower case "c" signify 2'-deoxy-2'-fluoro U and 2'-deoxy-2'-fluoro C residues. As used herein, the RPI# corresponds to the RPI numbers listed in WO 03/074654.

In another embodiment, the siRNA has the following construct, referred to herein as "Construct B": Chemically modified siRNA construct comprising 2'-deoxy-2'-fluoro pyrimidine nucleotides and purine ribonucleotides in which the sense strand of the siRNA is further modified with 5' and 3'-terminal inverted deoxyabasic caps, and the antisense strand comprises a 3'-terminal phosporothioate internucleotide linkage RPI#31310/31311, wherein 31310 has the nucleic acid sequence BAAAGCCACUCCACUCUCUUTTB (SEQ ID NO: 122); 31311 has the nucleic acid sequence AAGAGAGUGGAGUGGCUUUTST (SEQ ID NO: 123), and wherein B is a nucleosidic base such as adenine, guanine, uracil, cytosine, thymine, 2-aminoadenosine, 5-methylcytosine, 2,6-diaminopurine, or any other non-naturally occurring base that can be complementary or non-complementary to target RNA or a non- nucleosidic base such as phenyl, naphthyl, 3-nitropyrrole, 5-nitroindole, nebularine, pyridone, pyridinone, or any other non-naturally occurring universal base that can be complementary or non-complementary to target RNA. *(See e.g.,* PCT Publication No. WO 03/074654).

Suitable siRNA that target BCL2 include, e.g., siRNA molecules having the following sequences: AACAUCGCCCUGUGGAUGACU (SEQ ID NO:124), AAGTACATCCAGTATCAGATG (SEQ ID NO:125), GCCUUUGUGGAACUGUACGG (SEQ ID NO:126) and UGUGGAUGACUGAGUACCUGA (SEQ ID NO:127) *(See e.g.,* Chawla-Sarkar, et al., Cell Death and Differentiation, vol. 11:915-23 (2004) and Wacheck et al., Oligonucleotides, vol. 13(5):393-400 (2003)). In another embodiment of the invention, the siRNA is specific for BCL2 and has the following construct: RNA + 3'-terminal dithymidine: RPI#30998/31074, wherein 30998 has the nucleic acid sequence GGGAUGAUCAACAGGGUAGTT (SEQ ID NO:128); and 31074 has the nucleic acid sequence CUACCCUGUUGAUCAUCCCTT (SEQ ID NO: 129). Other suitable BCL2-specific siRNA molecules include, *e.g.,* BGG GAu GAu cAA cAG GGu AGT TB (SEQ ID NO:130); cuA ccc uGu uGA ucA ucc cTs T (SEQ ID NO:131); B *GGGAuGAucAAcAGGGuAGTT* B (SEQ ID NO: 132); cuAcccuGuuGAucAucccTsT (SEQ ID NO: 133); B *GAuGGGAcAAcuAGuAGGG*TT B (SEQ ID NO: 169); cccu*A*cu*AG*uu*G*uccc*A*ucTsT (SEQ ID NO: 170); B GAuGGGAcAAcuAGuAGGGTT B (SEQ ID NO: 134) and cccuAcuAGuuGucccAucTsT (SEQ ID NO: 135), where the nucleotides presented in uppercase are ribonucleotides, lower case nucleotides "u" and lower case "c" signify 2'-deoxy-2'-fluoro U and 2'-deoxy-2'-fluoro C residues, T is thymidine, B is an inverted deoxy abasic residue, s is a phosphorthioate linkage, A signifies a deoxy Adenosine residue and G represents a deoxy'Guanosine residue. *(See e.g.,* PCT Publication No. WO 03/074654).

In another embodiment, the siRNA includes 2'deoacy-2'-fluoro pyrimidine and purine ribonucleotides. The sense strand is further modified to include 5' and 3'-terminal phosphorothiote internucleotide linkage: RPI#31370/31371, wherein 31370 has the nucleic acid sequence BGAUGGGACAACUAGUAGGGTTB (SEQ ID NO: 136); and 31371 has the nucleic acid sequence CCCUACUAGUUGUCCCAUCTST (SEQ ID NO:137). *(See e.g.,* PCT Publication No. WO 03/074654).

In another embodiment of the invention, the siRNA is specific for another target, including, for example, NFkB. For example, siRNA that target NF-κB p50 include an siRNA having the sequence: TATTAGAGCAACCTAAACA (SEQ ID NO:138).

Suitable siRNA that target cMyc include, e.g., the following sequences: AGAGGGUCAAGUUGGACAGTT (SEQ ID NO: 139); CUGUCCAACUUGACCCUCUTT (SEQ ID NO: 140); GCAGAGGAGCAAAAGCUCATT (SEQ ID NO: 141); UCAAGACUCAGCCAAGGUUTT (SEQ ID NO: 142); CGGAACUCUUGUGCGUAAGTT (SEQ ID NO: 143); CUUACGCACAAGAGUUCCGTT (SEQ ID NO: 144); AACCUUGGCUGAGUCUUGATT (SEQ ID NO: 145); and UCAAGACUCAGCCAAGGUUTT (SEQ ID NO: 146) *(See e.g.,* PCT Publication No. WO 03/074654).

In one embodiment of the invention, the siRNA is specific for c-Myc and has a construct selected from the following constructs: RNA + 3'-terminal dT cap RPI 30993/31069, wherein 30993 has the nucleic acid sequence AGAGGGUCAAGUUGGACAGTT (SEQ ID NO: 128); 31069 has the nucleic acid sequence CUGUCCAACUUGACCCUCUTT (SEQ ID NO: 129); RNA + 3'-terminal dT cap RPI 30995/31071, wherein 30995 has the nucleic acid sequence CGGAACUCUUGUGCGUAAGTT (SEQ ID NO: 136); 31071 has the nucleic acid sequence CUUACGCACAAGAGUUCCGTT (SEQ ID NO:137); or RNA + 3'-terminal dT cap RPI 30996/31072, wherein 30996 has the nucleic acid sequence AACCUUGGCUGAGUCUUGATT (SEQ ID NO: 147); 31072 has the nucleic acid sequence UCAAGACUCAGCCAAGGUUTT (SEQ ID NO:148). (See e.g., PCT Publication No. WO 03/074654).

In another embodiment, the siRNA is specific for cyclin D1 and has the following construct: RNA + 3'-dT cap: RPI#31009/31085, wherein 31009 = ACACUUCCUCUCCAAAAUGTT (SEQ ID NO: 149) and 31085 = CAUUUUGGAGAGGAAGUGUTT (SEQ ID NO:150). *(See e.g.,* PCT Publication No. WO 03/074654, hereby incorporated by reference in its entirety).

Thus, in one embodiment, the therapeutically active oligonucleotide is an siRNA oligonucleotide that comprises or consists of one of the RNA sequences disclosed in the references cited above (i.e., *Chawla-Sarkar et al.* (2004); Wacheck *et al.* (2003); Liao et *al.* (2005); and PCT Publication No. WO 03/074654). Materials and methods for mediating intracellular delivery of an siRNA oligonucleotide having any one of the sequences disclosed in these references constitute specific embodiments of the present invention for which protection is hereby sought.

### Construct Design

In their simplest embodiment the constructs of this invention include two linked functional units - a first unit which enables delivery to the interior of a cell and is an aptamer, and a second unit that confers a phenotypic response and is a therapeutic siRNA. The two units are covalently connected, for example, by a bond that is designed to be likely to break in the interior of the cell.

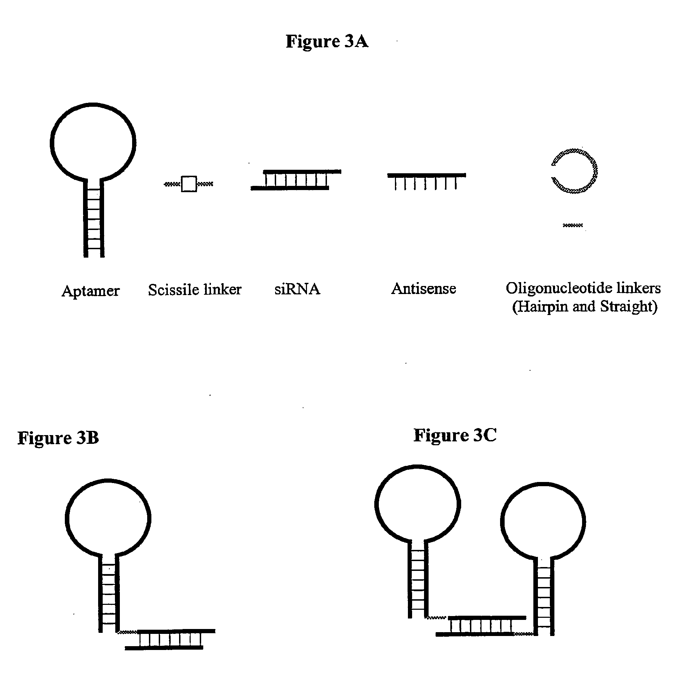

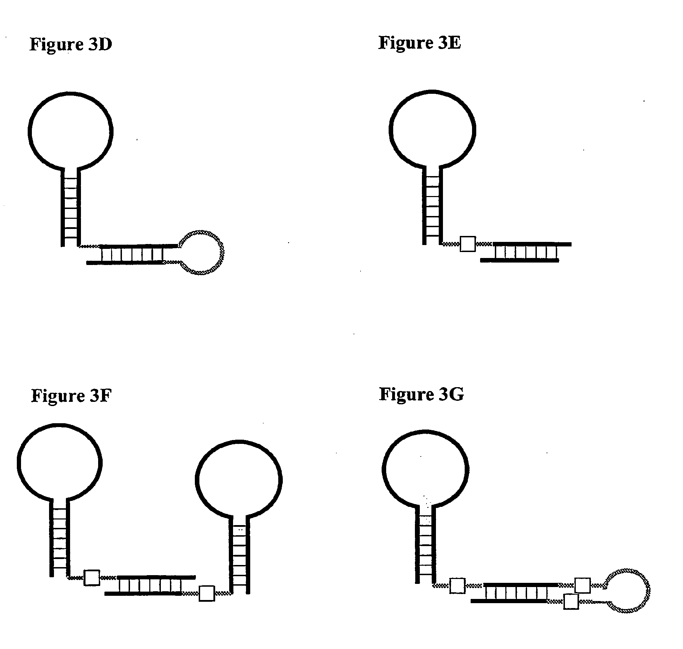

Examples of design strategies according to the invention are shown in Figures 3B-3I (the legend for these figures is shown in Figure 3A). The scissile linkages shown in Figure 3A include, for example, nucleotides, disulfide bonds, and esters.

Domain II is linked to Domain I in a variety of configurations. For example, in on e embodiment, Domain II is conjugated to either the 5' or 3' terminus of Domain I. These two domains are conjugated together in a separate chemical step, e.g., using combination chemistry techniques or disulfide chemistry techniques. In another embodiment, Domain I and Domain II are synthesized as part of a larger nucleic acid molecule, such that the longer nucleic acid molecule folds upon itself, such that Domain I and Domain II are brought into a desired proximity and/or configuration.

In the construct designs shown below in Table 3, all compositions are in the "rRfy" form, which includes ribonucleotide purines and 2' fluorinated pyrimidines. The constructs described below are designed to be transcribed. In the following constructs, three design strategies are applied to combinations of two siRNA constructs (which are specific for EGFR and BCL2) and one aptamer sequence (which is specific for PSMA).

**Table 3**

| | |
|---|---|
| **Design elements** | |
| | |
| PSMA Aptamer (A9) | |
| GGAGGACCGAAAAAGACCUGACUUCUAUACUAAGUCUACGUUCCUCC (SEQ ID NO:151), | |
| | |
| EGF receptor siRNA | |
| RPI#31300 Bcc AAG ucc uAc AGA cuc cAT TB (SEQ ID NO:152) | |
| RPI#31301 uGG AGu cuG uAG GAc uuG GTs T (SEQ ID NO:153) | |
| | |
| EGFR | |
| 5' CCAAGUCCUACAGACUCCAUU (SEQ ID NO:154) (+) | |
| 3'UUGGUUCAGGAUGUCUGAGGU (SEQ m NO:155) (-) | |
| (5'UGGAGUCUGUAGGACUUGGUU) (SEQ ID NO:156) | |
| | |
| BCL2 siRNA | |
| 31368 BGG GAu GAu cAA cAG GGu AGT TB (SEQ ID NO:157) | |
| 31369 cuA ccc uGu uGA ucA ucc cTs T (SEQ ID NO:158) | |
| | |
| BCL2 | |
| 5' GGGAUGAUCAACAGGGUAGUU (SEQ ID NO:159) (+) | |
| 3'UUCCCUACUAGUUGUCCCAUC (SEQ ID NO:160) (-) | |
| (5'CUACCCUGUUGAUCAUCCCUU) (SEQ ID NO:161) | |
| Straight linker | |
| AAAAA | |
| | |
| Hairpin linker | |
| GAAAAA | |
| | |
| **Construct Design 1** (Figures 3C, 3F) | |
| Aptamer-linker-siRNA | |
| | SiRNA-linker-Aptamer |
| | |
| 1-EGFR(+) (combine with 1-EFGR(-)) | |
| GGAGGACCGAAAAAGACCUGACUUCUAUACUAAGUCUACGUUCCUCC AAAAA CCAAGUCCUACAGACUCCAUU (SEQ ID NO:162) | |
| | |
| 1-EFGR(-)(combine with 1-EFGR(+)) | |
| GGAGGACCGAAAAAGACCUGACUUCUAUACUAAGUCUACGUUCCUCC AAAAA UGGAGUCUGUAGGACUUGGUU (SEQ ID NO:163) | |
| | |
| 1-BCL2(+)(combine with 1-BCL2(-)) | |
| GGAGGACCGAAAAAGACCUGACUUCUAUACUAAGUCUACGUUCCUCC AAAAA GGGAUGAUCAACAGGGUAGUU (SEQ ID NO:164) | |
| | |
| 1-BCL2(-)(combine with 1-BCL2(+)) | |
| GGAGGACCGAAAAAGACCUGACUUCUAUACUAAGUCUACGUUCCUCC AAAAA CUACCCUGUUGAUCAUCCCUU (SEQ ID NO:165) | |
| | |
| **Construct Design 2** (Figures 3D, 3G) | |
| Aptamer-linker-siRNA-hairpin-linker-siRNA | |
| | |
| 2-EFGR | |
| GGAGGACCGAAAAAGACCUGACUUCUAUACUAAGUCUACGUUCCUCC CCAAGUCCUACAGACUCCAUU GAAAAA UGGAGUCUGUAGGACUUGGUU (SEQ ID NO:166) | |
| | |
| 2-BCL2 | |
| GGAGGACCGAAAAAGACCUGACUUCUAUACUAAGUCUACGUUCCUCC GGGAUGAUCAACAGGGUAGUU GAAAAA CUACCCUGUUGAUCAUCCCUU (SEQ ID NO:167) | |
| | |
| **Construct Design 3** (Figures 3B, 3E) | |
| Aptamer-linker-siRNA | |
| | siRNA |
| 3-BCL2(+)(combine with 1-BCL2(-)) | |
| GGGAUGAUCAACAGGGUAGUU (SEQ ID NO:168) | |

### Pharmaceutical Compositions

The invention also includes pharmaceutical compositions containing aptamer molecules. In some embodiments, the compositions are suitable for internal use and include an effective amount of a pharmacologically active compound of the invention, alone or in combination, with one or more pharmaceutically acceptable carriers. The compounds are especially useful in that they have very low, if any toxicity.

Compositions of the invention can be used to treat or prevent a pathology, such as a disease or disorder, or alleviate the symptoms of such disease or disorder in a patient. For example, compositions of the invention can be used to treat or prevent a pathology associated with inflammation. Compositions of the invention are useful for administration to a subject suffering from, or predisposed to, a disease or disorder which is related to or derived from a target to which the aptamers specifically bind.

For example, the target is a protein involved with inflammation, for example, the target protein causes or contributes to inflammation.

Compositions of the invention can be used in a method for treating a patient having a pathology. The method involves administering to the patient a composition comprising aptamers that bind a target (e.g., a protein) involved with the pathology, so that binding of the composition to the target alters the biological function of the target, thereby treating the pathology.

The patient having a pathology, *e.g.* the patient treated by the methods of this invention can be a mammal, or more particularly, a human.

In practice, the compounds or their pharmaceutically acceptable salts, are administered in amounts which will be sufficient to exert their desired biological activity.

For instance, for oral administration in the form of a tablet or capsule *(e.g.,* a gelatin capsule), the active drug component can be combined with an oral, non-toxic pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include starch, magnesium aluminum silicate, starch paste, gelatin, methylcellulose, sodium carboxymethylcellulose and/or polyvinylpyrrolidone, natural sugars such as glucose or beta-lactose, corn sweeteners, natural and synthetic gums such as acacia, tragacanth or sodium alginate, polyethylene glycol, waxes and the like. Lubricants used in these dosage forms include sodium oleate, sodium stearate, magnesium stearate, sodium benzoate, sodium acetate, sodium chloride, silica, talcum, stearic acid, its magnesium or calcium salt and/or polyethyleneglycol and the like. Disintegrators include, without limitation, starch, methyl cellulose, agar, bentonite, xanthan gum starches, agar, alginic acid or its sodium salt, or effervescent mixtures, and the like. Diluents, include, *e.g.,* lactose, dextrose, sucrose, mannitol, sorbitol, cellulose and/or glycine.

Injectable compositions are preferably aqueous isotonic solutions or suspensions, and suppositories are advantageously prepared from fatty emulsions or suspensions. The compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. The compositions are prepared according to conventional mixing, granulating or coating methods, respectively, and contain about 0.1 to 75%, preferably about 1 to 50%, of the active ingredient.

The compounds of the invention can also be administered in such oral dosage forms as timed release and sustained release tablets or capsules, pills, powders, granules, elixirs, tinctures, suspensions, syrups and emulsions.

Liquid, particularly injectable compositions can, for example, be prepared by dissolving, dispersing, etc. The active compound is dissolved in or mixed with a pharmaceutically pure solvent such as, for example, water, saline, aqueous dextrose, glycerol, ethanol, and the like, to thereby form the injectable solution or suspension. Additionally, solid forms suitable for dissolving in liquid prior to injection can be formulated. Injectable compositions are preferably aqueous isotonic solutions or suspensions. The compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances.

The compounds of the present invention can be administered in intravenous (both bolus and infusion), intraperitoneal, subcutaneous or intramuscular form, all using forms well known to those of ordinary skill in the pharmaceutical arts. Injectables can be prepared in conventional forms, either as liquid solutions or suspensions.

Parental injectable administration is generally used for subcutaneous, intramuscular or intravenous injections and infusions. Additionally, one approach for parenteral administration employs the implantation of a slow-release or sustained-released systems, which assures that a constant level of dosage is maintained, according to U.S. Pat. No. 3,710,795.

Furthermore, preferred compounds for the present invention can be administered in intranasal form via topical use of suitable intranasal vehicles, or via transdermal routes, using those forms of transdermal skin patches well known to those of ordinary skill in that art. To be administered in the form of a transdermal delivery system, the dosage administration will, of course, be continuous rather than intermittent throughout the dosage regimen. Other preferred topical preparations include creams, ointments, lotions, aerosol sprays and gels, wherein the concentration of active ingredient would range from 0.01% to 15%, w/w or w/v.

For solid compositions, excipients include pharmaceutical grades of mannitol, lactose, starch, magnesium stearate, sodium saccharin, talcum, cellulose, glucose, sucrose, magnesium carbonate, and the like may be used. The active compound defined above, may be also formulated as suppositories using for example, polyalkylene glycols, for example, propylene glycol, as the carrier. In some embodiments, suppositories are advantageously prepared from fatty emulsions or suspensions.

The compounds of the present invention can also be administered in the form of liposome delivery systems, such as small unilamellar vesicles, large unilamellar vesicles and multilamellar vesicles. Liposomes can be formed from a variety of phospholipids, containing cholesterol, stearylamine or phosphatidylcholines. In some embodiments, a film of lipid components is hydrated with an aqueous solution of drug to a form lipid layer encapsulating the drug, as described in U.S. Pat. No. 5,262,564. For example, the aptamer molecules described herein can be provided as a complex with a lipophilic compound or non-immunogenic, high molecular weight compound constructed using methods known in the art. An example of nucleic-acid associated complexes is provided in US Patent No. 6,011,020.

The compounds of the present invention may also be coupled with soluble polymers as targetable drug carriers. Such polymers can include polyvinylpyrrolidone, pyran copolymer, polyhydroxypropyl-methacrylamide-phenol, polyhydroxyethylaspanamidephenol, or polyethyleneoxidepolylysine substituted with palmitoyl residues. Furthermore, the compounds of the present invention may be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates and cross-linked or amphipathic block copolymers of hydrogels.

If desired, the pharmaceutical composition to be administered may also contain minor amounts of non-toxic auxiliary substances such as wetting or emulsifying agents, pH buffering agents, and other substances such as for example, sodium acetate, triethanolamine oleate, etc.

The dosage regimen utilizing the compounds is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician or veterinarian can readily determine and prescribe the effective amount of the drug required to prevent, counter or arrest the progress of the condition.

Oral dosages of the present invention, when used for the indicated effects, will range between about 0.05 to 1000 mg/day orally. The compositions are preferably provided in the form of scored tablets containing 0.5, 1.0, 2.5, 5.0, 10.0, 15.0, 25.0, 50.0, 100.0, 250.0, 500.0 and 1000.0 mg of active ingredient. Effective plasma levels of the compounds of the present invention range from 0.002 mg to 50 mg per kg of body weight per day.

Compounds of the present invention may be administered in a single daily dose, or the total daily dosage may be administered in divided doses of two, three or four times daily.

Citation of publications and patent documents is not intended as an admission that any is pertinent prior art, nor does it constitute any admission as to the contents or date of the same. The invention having now been described by way of written description, those of skill in the art will recognize that the invention can be practiced in a variety of embodiments and that the foregoing description and examples below are for purposes of illustration and not limitation of the claims that follow.
The following numbered paragraphs contain statements of broad combinations of the inventive technical features herein disclosed:-
1. A therapeutic nucleic acid composition comprising a delivery aptamer sequence and a therapeutic oligonucleotide sequence linked by a linking moiety.
2. The therapeutic nucleic acid composition of paragraph 1, wherein the therapeutic oligonucleotide sequence is selected from the group consisting of a CpG oligonucleotide, an siRNA oligonucleotide, an antisense oligonucleotide, a ribozyme, an aptamer, and a nucleic acid decoy.
3. The therapeutic nucleic acid composition of paragraph 1, wherein the therapeutic oligonucleotide is an siRNA oligonucleotide covalently attached to the delivery aptamer sequence through a 5'-3' phosphodiester linkage.
4. The therapeutic nucleic acid composition of paragraph 3, wherein coding strand of the siRNA oligonucleotide is covalently attached to the delivery aptamer through a 5'-3' phosphodiester linkage at the 5'-end of the delivery aptamer.
5. The therapeutic nucleic acid composition of paragraph 3, wherein coding strand of the siRNA oligonucleotide is covalently attached to the delivery aptamer through a 5'-3' phosphodiester linkage at the 3'-end of the delivery aptamer.
6. The therapeutic nucleic acid composition of paragraph 3, wherein the coding and noncoding strands of the siRNA oligonucleotide comprise a contiguous sequence covalently attached to the delivery aptamer through a 5'-3' phosphodiester linkage to either the 5'- or 3'-end of the delivery aptamer.
7. The therapeutic nucleic acid composition of paragraph 1, wherein the therapeutic oligonucleotide is an siRNA oligonucleotide covalently attached to the delivery aptamer sequence through a oligonucleotide linker having a length of ten nucleotides or less.
8. The therapeutic nucleic acid composition of paragraph 1, wherein the therapeutic oligonucleotide is an siRNA oligonucleotide specific for a target selected from the group consisting of proliferating cell nuclear antigen (PCNA), Androgen Receptor, BCL2, NFkB, VEGFR1, VEGFR2, VEGFR3, HER2/neu, c-Myc, REL-A, PTP1B, BACE, CHK1, PKC-alpha, EGFR, CHK-1, ERG2, Cyclin D1, CCND1 P, KCa, RAF1, MAPK1, MAPK8, MYB, KRAS2, KDR, IKKg, hTR, HRAS, FOS, GAB2, FLT1, EZH2 and CDK2.
9. The therapeutic nucleic acid composition of paragraph 1, wherein the linking moiety is a nucleic acid moiety, a PNA moiety, a peptidic moiety, a disulfide bond or a polyethylene glycol moiety.
10. The therapeutic nucleic acid composition of paragraph 1, wherein the delivery aptamer sequence binds to a target selected from the group consisting of a tumor antigen, a growth factor receptor, a cytokine receptor, a chemokine receptor, a G-protein coupled receptor, neurotensin (NTS-1) protein, a cytokine-cytokine receptor complex, and a viral coat protein.
11. The therapeutic nucleic acid composition of paragraph 1, wherein the delivery aptamer sequence binds to a target selected from the group consisting of PSMA, Her2/Neu, EGFRI-III, MUC-1, CD4, cMET, CEA, CD6, CD19, CD22, CD23, CD33, CD44v6, CD56, VEGFRI, VEGFRII, NTS-1 protein, gp120 coat protein, gp41 fusion peptide, TENB2, HER2/neu (erb2), EGF receptor vIII, Cripto-1, and Alpha(v)beta(3/5).
12. The therapeutic nucleic acid composition of paragraph 1 further comprising a modified nucleotide.
13. The therapeutic nucleic acid composition of paragraph 1 further comprising an abasic residue.
14. The therapeutic nucleic acid composition of paragraph 1 further comprising a polyalkylene glycol residue.
15. A therapeutic nucleic acid composition comprising a nucleic acid sequence selected from the group consisting of SEQ ID NO:1-169 and SEQ ID NO: 170.
16. A method of modulating the intracellular delivery of a therapeutic agent in a subject comprising administering to said subject the therapeutic nucleic acid composition of claim 1.
17. A therapeutic nucleic acid composition comprising a delivery aptamer sequence, a therapeutic oligonucleotide, and a linking moiety, wherein the linking moiety is not a nucleic acid molecule.
18. The therapeutic nucleic acid composition of paragraph 17, wherein the linking moiety comprises a polyalkylene glycol.
19. The therapeutic nucleic acid composition of paragraph 18, wherein the linking moiety comprises polyethylene glycol (PEG).
20. The therapeutic nucleic acid composition of paragraph 19, wherein the delivery aptamer sequence and the therapeutic oligonucleotide are linked by the PEG linking moiety, and further wherein the primary structure of the therapeutic nucleic acid composition comprises a linear arrangement in which the delivery aptamer is linked to a first terminus of the PEG linking moiety and the therapeutic oligonucleotide is linked to a second terminus of the PEG linking moiety.

## Claims

1. A bi-functional therapeutic oligonucleotide comprising a delivery aptamer covalently linked to a therapeutic siRNA for use in the treatment or prevention of disease, wherein the delivery aptamer binds to a target that is a cell surface protein or tumor antigen.

2. The bi-functional therapeutic oligonucleotide for use in the treatment or prevention of disease of claim 1 wherein the therapeutic oligonucleotide consists of a delivery aptamer and a therapeutic siRNA.

3. The bi-functional therapeutic oligonucleotide for use in the treatment or prevention of disease of claim 1 or claim 2 wherein the siRNA is covalently attached to the delivery aptamer through a 5'-3' phosphodiester linkage.

4. The bi-functional therapeutic oligonucleotide for use in the treatment or prevention of disease of any one of claims 1 to 3 wherein the coding strand of the siRNA is covalently attached to the delivery aptamer through a 5'-3' phosphodiester linkage at the 5'- or 3'- end of the delivery aptamer.

5. The bi-functional therapeutic oligonucleotide for use in the treatment or prevention of disease of any one of claims 1 to 4 wherein the coding and noncoding strands of the siRNA comprise a contiguous sequence covalently attached to the delivery aptamer through a 5'-3' phosphodiester linkage to either the 5'- or 3'- end of the delivery aptamer.

6. The bi-functional therapeutic oligonucleotide for use in the treatment or prevention of disease according to any one of the preceding claims wherein the siRNA is covalently attached to the delivery aptamer through an oligonucleotide linker having a length of ten nucleotides or less.

7. The bi-functional therapeutic oligonucleotide for use in the treatment or prevention of disease of any one of claims 1 to 6 wherein the linking moiety is a nucleic acid moiety, a PNA moiety, a peptidic moiety, a disulfide bond or a polyethylene glycol moiety.

8. The bi-functional therapeutic oligonucleotide for use in the treatment or prevention of disease of any one of the preceding claims wherein treatment of the disease involves modulating the intracellular delivery of the therapeutic siRNA in the patient by binding of the aptamer to its target in order to mediate the intracellular delivery of the therapeutic siRNA.

9. The bi-functional therapeutic oligonucleotide for use in the treatment or prevention of disease according to any one of the preceding claims wherein the delivery aptamer binds to a target selected from the group consisting of: PSMA. Her2/Neu, EGFRI-III, MUC-1, CD4, cMET, CEA, CD6, CD19, CD22, CD23, CD33, CD44v6, CD56, VEGFRI, VEGFRII, NTS-1 protein, gp120 coat protein, gp41 fusion peptide, TENB2, HER2/neu (erb2), EGF receptor vIII, Cripto-1, and Alpha(v)beta(3/5).

10. The bi-functional therapeutic oligonucleotide for use in the treatment or prevention of disease of any one of claims 1 to 9 wherein the delivery aptamer binds to PSMA.

11. The bi-functional therapeutic oligonucleotide for use in the treatment or prevention of disease of any one of claims 1 to 10 wherein the delivery aptamer is A9 (SEQ ID No.1) or A10 (SEQ ID No.2).

12. The bi-functional therapeutic oligonucleotide for use in the treatment or prevention of disease of any one of the preceding claims wherein the therapeutic siRNA is specific for a target selected from the group consisting of: proliferating cell nuclear antigen (PCNA), Androgen Receptor, BCL2, NFkB, VEGFRI, VEGFR2, VEGFR3, HER2/neu, c-Myc, REL-A, PTP1B, BACE, CHK1, PKC-alpha, EGFR, CHK-1, ERG2, Cyclin DI, CCND1 P, KCa, RAF1, MAPK1, MAPK8, MYB, KRAS2, KDR, IKKg, hTR, HRAS, FOS, GAB2, FLT1, EZH2 and CDK2.

13. The bi-functional therapeutic oligonucleotide for use in the treatment or prevention of disease of any one of the preceding claims wherein the therapeutic siRNA is specific for BCL2.

14. The bi-functional therapeutic oligonucleotide for use in the treatment or prevention of disease of any one of the preceding claims wherein the disease is cancer.

15. A bi-functional therapeutic oligonucleotide comprising a delivery aptamer covalently linked to a therapeutic siRNA, wherein the delivery aptamer binds to a target that is a cell surface protein or tumor antigen.

16. The bi-functional therapeutic oligonucleotide according to claim 15 which consists of a delivery aptamer and a therapeutic siRNA.

17. A bi-functional therapeutic oligonucleotide according to any one of claims 15 or 16 wherein the siRNA is covalently attached to the delivery aptamer through a 5'-3' phosphodiester linkage.

18. A bi-functional therapeutic oligonucleotide according to any one of claims 15 to 17 wherein the coding strand of the siRNA is covalently attached to the delivery aptamer through a 5'-3' phosphodiester linkage at the 5'- or 3'- end of the delivery aptamer.

19. A bi-functional therapeutic oligonucleotide according to any one of claims 15 to 18 wherein the coding and noncoding strands of the siRNA comprise a contiguous sequence covalently attached to the delivery aptamer through a 5'-3' phosphodiester linkage to either the 5'- or 3'- end of the delivery aptamer.

20. A bi-functional therapeutic oligonucleotide according to any one of claims 15 to 19 wherein the siRNA is covalently attached to the delivery aptamer through an oligonucleotide linker having a length of ten nucleotides or less.

21. A bi-functional therapeutic oligonucleotide according to any one of claims 15 to 20 wherein the linking moiety is a nucleic acid moiety, a PNA moiety, a peptidic moiety, a disulfide bond or a polyethylene glycol moiety.

22. A bi-functional therapeutic oligonucleotide according to any one of claims 15 to 21 for use in the treatment of cancer.

23. A bi-functional therapeutic oligonucleotide according to claim 22 wherein the treatment involves modulating the intracellular delivery of the therapeutic siRNA in the patient by binding of the aptamer to its target in order to mediate the intracellular delivery of the therapeutic siRNA.

24. A bi-functional therapeutic oligonucleotide according to any one of claims 15 to 23 wherein the delivery aptamer binds to a target selected from the group consisting of: PSMA, Her2/Neu, EGFRI-III, MUC-1, CD4, cMET, CEA, CD6, CD19, CD22, CD23, CD33, CD44v6, CD56, VEGFRI, VEGFRII, NTS-1 protein, gp120 coat protein, gp41 fusion peptide, TENB2, HER2/neu (erb2), EGF receptor vIII, Cripto-1, and Alpha(v)beta(3/5).

25. A bi-functional therapeutic oligonucleotide according to any one of claims 15 to 23 wherein the delivery aptamer binds to PSMA.

26. A bi-functional therapeutic oligonucleotide according to any one of claims 15 to 25 wherein the delivery aptamer is A9 (SEQ ID No.1) or A10 (SEQ ID No.2).

27. A bi-functional therapeutic oligonucleotide according to any one of claims 15 to 26 wherein the therapeutic siRNA oligonucleotide is specific for a target selected from proliferating cell nuclear antigen (PCNA), Androgen Receptor, BCL2, NFkB, VEGFRI, VEGFR2, VEGFR3, HER2/neu, c-Myc, REL-A, PTP1B, BACE, CHK1, PKC-alpha, EGFR, CHK-I, ERG2, Cyclin DI, CCND1 P, KCa, RAF1, MAPK1, MAPK8, MYB, KRAS2, KDR, IKKg, hTR, HRAS, FOS, GAB2, FLT1, EZH2 and CDK2.

28. A bi-functional therapeutic oligonucleotide according to any one of claims 15 to 27 wherein the therapeutic siRNA oligonucleotide sequence is specific for BCL2.

## Patentansprüche

1. Bifunktionelles therapeutisches Oligonucleotid, das ein Zufuhraptamer kovalent an eine therapeutische siRNA gebunden umfasst, zur Verwendung bei der Behandlung oder Vorbeugung einer Erkrankung, wobei das Zufuhraptamer an ein Target bindet, das ein Zelloberflächenprotein oder Tumorantigen ist.

2. Bifunktionelles therapeutisches Oligonucleotid zur Verwendung bei der Behandlung oder Vorbeugung einer Erkrankung nach Anspruch 1, wobei das therapeutische Oligonucleotid aus einem Zufuhraptamer und einer therapeutischen siRNA besteht.

3. Bifunktionelles therapeutisches Oligonucleotid zur Verwendung bei der Behandlung oder Vorbeugung einer Erkrankung nach Anspruch 1 oder Anspruch 2, wobei die siRNA über eine 5'-3'-Phosphordiesterbindung kovalent an das Zufuhraptamer gebunden ist.

4. Bifunktionelles therapeutisches Oligonucleotid zur Verwendung bei der Behandlung oder Vorbeugung einer Erkrankung nach einem der Ansprüche 1 bis 3, wobei der kodierende Strang der siRNA über eine 5'-3'-Phosphodiesterbindung am 5'- oder 3'-Ende des Zufuhraptamers kovalent an das Zufuhraptamer gebunden ist.

5. Bifunktionelles therapeutisches Oligonucleotid zur Verwendung bei der Behandlung oder Vorbeugung einer Erkrankung nach einem der Ansprüche 1 bis 4, wobei der kodierende und nichtkodierende Strang der siRNA eine zusammenhängende Sequenz über eine 5'-3'-Phosphodiesterbindung am 5'- oder 3'-Ende des Zufuhraptamers kovalent an das Zufuhraptamer gebunden umfasst.

6. Bifunktionelles therapeutisches Oligonucleotid zur Verwendung bei der Behandlung oder Vorbeugung einer Erkrankung nach einem der vorangegangenen Ansprüche, wobei die siRNA über einen Oligonucleotidlinker mit einer Länge von zehn Nucleotiden oder weniger kovalent an das Zufuhraptamer gebunden ist.

7. Bifunktionelles therapeutisches Oligonucleotid zur Verwendung bei der Behandlung oder Vorbeugung einer Erkrankung nach einem der Ansprüche 1 bis 6, wobei die Bindungsgruppierung eine Nucleinsäuregruppierung, eine PNA-Gruppierung, eine Peptidgruppierung, eine Disulfidbindung oder eine Polyethylenglykolgruppierung ist.

8. Bifunktionelles therapeutisches Oligonucleotid zur Verwendung bei der Behandlung oder Vorbeugung einer Erkrankung nach einem der vorangegangenen Ansprüche, wobei die Behandlung der Erkrankung das Modulieren der intrazellulären Zufuhr der therapeutischen siRNA im Patienten durch Bindung des Aptamers an sein Target umfasst, um die intrazelluläre Zufuhr der therapeutischen siRNA zu vermitteln.

9. Bifunktionelles therapeutisches Oligonucleotid zur Verwendung bei der Behandlung oder Vorbeugung einer Erkrankung nach einem der vorangegangenen Ansprüche, wobei das Zufuhraptamer an ein Target bindet, das aus folgender Gruppe ausgewählt ist: PSMA, Her2/Neu, EGFRI-III, MUC-1, CD4, cMET, CEA, CD6, CD19, CD22, CD23, CD33, CD44v6, CD56, VEGFRI, VEGFRII, NTS-1-Protein, gp120-Hüll-protein, gp41-Fusionspeptid, TENB2, HER2/neu (erb2), EGF-Rezeptor viii, Cripto-1 und Alpha(v)beta(3/5).

10. Bifunktionelles therapeutisches Oligonucleotid zur Verwendung bei der Behandlung oder Vorbeugung einer Erkrankung nach einem der Ansprüche 1 bis 9, wobei das Zufuhraptamer an PSMA bindet.

11. Bifunktionelles therapeutisches Oligonucleotid zur Verwendung bei der Behandlung oder Vorbeugung einer Erkrankung nach einem der Ansprüche 1 bis 10, wobei das Zufuhraptamer A9 (Seq.-ID Nr. 1) oder A10 (Seq.-ID Nr. 2) ist.

12. Bifunktionelles therapeutisches Oligonucleotid zur Verwendung bei der Behandlung oder Vorbeugung einer Erkrankung nach einem der vorangegangenen Ansprüche, wobei die therapeutische siRNA spezifisch für ein Target ist, das aus folgender Gruppe ausgewählt ist: Proliferating-Cell-Nuclear-Antigen (PCNA), Androgenrezeptor, BCL2, NFkB, VEGFRI, VEGFR2, VEGFR3, HER2/neu, c-Myc, REL-A, PTP1B, BACE, CHK1, PKC-α, EGFR, CHK-I, ERG2, Cyclin DI, CCND1 P, KCa, RAF1, MAPK1, MAPK8, MYB, KRAS2, KDR, IKKg, hTR, HRAS, FOS, GAB2, FLT1, EZH2 und CDK2.

13. Bifunktionelles therapeutisches Oligonucleotid zur Verwendung bei der Behandlung oder Vorbeugung einer Erkrankung nach einem der vorangegangenen Ansprüche, wobei die therapeutische siRNA spezifisch für BCL2 ist.

14. Bifunktionelles therapeutisches Oligonucleotid zur Verwendung bei der Behandlung oder Vorbeugung einer Erkrankung nach einem der vorangegangenen Ansprüche, wobei die Erkrankung Krebs ist.

15. Bifunktionelles therapeutisches Oligonucleotid, das ein Zufuhraptamer kovalent an eine therapeutische siRNA gebunden umfasst, wobei das Zufuhraptamer an ein Target bindet, das ein Zelloberflächenprotein oder Tumorantigen ist.

16. Bifunktionelles therapeutisches Oligonucleotid nach Anspruch 15, das aus einem Zufuhraptamer und einer therapeutischen siRNA besteht.

17. Bifunktionelles therapeutisches Oligonucleotid nach einem der Ansprüche 15 oder 16, wobei die siRNA über eine 5'-3'-Phosphodiesterbindung kovalent an das Zufuhraptamer gebunden ist.

18. Bifunktionelles therapeutisches Oligonucleotid nach einem der Ansprüche 15 bis 17, wobei der kodierende Strang der siRNA über eine 5'-3'-Phosphodiesterbindung am 5'- oder 3'-Ende des Zufuhraptamers kovalent an das Zufuhraptamer gebunden ist.

19. Bifunktionelles therapeutisches Oligonucleotid nach einem der Ansprüche 15 bis 18, wobei der kodierende und nichtkodierende Strang der siRNA eine zusammenhängende Sequenz über eine 5'-3'-Phosphodiesterbindung am 5'- oder 3'-Ende des Zufuhraptamers kovalent an das Zufuhraptamer gebunden umfasst.

20. Bifunktionelles therapeutisches Oligonucleotid nach einem der Ansprüche 15 bis 19, wobei die siRNA über einen Oligonucleotidlinker mit einer Länge von zehn Nucleotiden oder weniger kovalent an das Zufuhraptamer gebunden ist.

21. Bifunktionelles therapeutisches Oligonucleotid nach einem der Ansprüche 15 bis 20, wobei die Bindungsgruppierung eine Nucleinsäuregruppierung, eine PNA-Gruppierung, eine Peptidgruppierung, eine Disulfidbindung oder eine Polyethylenglykolgruppierung ist.

22. Bifunktionelles therapeutisches Oligonucleotid nach einem der Ansprüche 15 bis 21 zur Verwendung bei der Behandlung von Krebs.

23. Bifunktionelles therapeutisches Oligonucleotid nach Anspruch 22, wobei die Behandlung das Modulieren der intrazellulären Zufuhr der therapeutischen siRNA im Patienten durch Bindung des Aptamers an sein Target umfasst, um die intrazelluläre Zufuhr der therapeutischen siRNA zu vermitteln.

24. Bifunktionelles therapeutisches Oligonucleotid nach einem der Ansprüche 15 bis 23, wobei das Zufuhraptamer an ein Target bindet, das aus folgender Gruppe ausgewählt ist: PSMA, Her2/Neu, EGFRI-III, MUC-1, CD4, cMET, CEA, CD6, CD19, CD22, CD23, CD33, CD44v6, CD56, VEGFRI, VEGFRII, NTS-1-Protein, gp120-Hüll-protein, gp41-Fusionspeptid, TENB2, HER2/neu (erb2), EGF-Rezeptor viii, Cripto-1 und Alpha(v)beta(3/5).

25. Bifunktionelles therapeutisches Oligonucleotid nach einem der Ansprüche 15 bis 23, wobei das Zufuhraptamer an PSMA bindet.

26. Bifunktionelles therapeutisches Oligonucleotid nach einem der Ansprüche 15 bis 25, wobei das Zufuhraptamer A9 (Seq.-ID Nr. 1) oder A10 (Seq.-ID Nr. 2) ist.

27. Bifunktionelles therapeutisches Oligonucleotid nach einem der Ansprüche 15 bis 26, wobei die therapeutische siRNA spezifisch für ein Target ist, das aus folgender Gruppe ausgewählt ist: Proliferating-Cell-Nuclear-Antigen (PCNA), Androgenrezeptor, BCL2, NFkB, VEGFRI, VEGFR2, VEGFR3, HER2/neu, c-Myc, REL-A, PTP1B, BACE, CHK1, PKC-α, EGFR, CHK-I, ERG2, Cyclin DI, CCND1 P, KCa, RAF1, MAPK1, MAPK8, MYB, KRAS2, KDR, IKKg, hTR, HRAS, FOS, GAB2, FLT1, EZH2 und CDK2.

28. Bifunktionelles therapeutisches Oligonucleotid nach einem der Ansprüche 15 bis 27, wobei die Oligonucleotidsequenz der therapeutischen siRNA für BCL2 spezifisch ist.

## Revendications

1. Oligonucléotide thérapeutique bifonctionnel comprenant un aptamère de délivrance, lié de manière covalente à un ARNsi thérapeutique pour utilisation dans le traitement ou la prévention d'une maladie, où l'aptamère de délivrance se lie à une cible qui est une protéine de surface de cellule ou un antigène de tumeur.

2. Oligonucléotide thérapeutique bifonctionnel pour utilisation dans le traitement ou la prévention de la maladie selon la revendication 1, où l'oligonucléotide thérapeutique est constitué d'un aptamère de délivrance et d'un ARNsi thérapeutique.

3. Oligonucléotide thérapeutique bifonctionnel pour utilisation dans le traitement ou la prévention de la maladie selon la revendication 1 ou la revendication 2, où l'ARNsi est attaché de manière covalente à l'aptamère de délivrance par une liaison de 5'-3' phosphodiester.

4. Oligonucléotide thérapeutique bifonctionnel pour utilisation dans le traitement ou la prévention de la maladie selon l'une quelconque des revendications 1 à 3, où le brin de codage du ARNsi est lié de manière covalente à l'aptamère de délivrance par une liaison de 5'-3' phosphodiester à l'extrémité 5' ou 3' de l'aptamère de délivrance.

5. Oligonucléotide thérapeutique bifonctionnel pour utilisation dans le traitement ou la prévention de la maladie selon l'une quelconque des revendications 1 à 4, où les brins de codage et de non-codage de l'ARNsi comprennent une séquence contiguë liée de manière covalente à l'aptamère de délivrance par une liaison de 5'-3' phosphodiester à l'une quelconque des extrémités 5' ou 3' de l'aptamère de délivrance.

6. Oligonucléotide thérapeutique bi-fonctionnel pour l'utilisation dans le traitement ou la prévention de la maladie selon l'une quelconque des revendications précédentes, où l'ARNsi est lié de manière covalente à l'aptamère de délivrance par un lieur d'oligonucléotide d'une longueur de dix nucléotides ou moins.

7. Oligonucléotide thérapeutique bi-fonctionnel pour utilisation dans le traitement ou la prévention de la maladie selon l'une quelconque des revendications 1 à 6, où le fragment de liaison est un fragment d'acide nucléique, un fragment ANP, un fragment peptidique, un lien de disulfure ou un fragment de polyéthylène glycol.

8. Oligonucléotide thérapeutique bi-fonctionnel pour utilisation dans le traitement ou la prévention de la maladie selon l'une quelconque des revendications précédentes, où le traitement de la maladie implique la modulation de la délivrance intracellulaire de l'ARNsi thérapeutique chez le patient en liant l'aptamère à sa cible afin de réguler la délivrance intracellulaire de l'ARNsi thérapeutique.

9. Oligonucléotide thérapeutique bi-fonctionnel pour utilisation dans le traitement ou la prévention de la maladie selon l'une quelconque des revendications précédentes, où l'aptamère de délivrance se lie à une cible sélectionnée dans le groupe consistant en: PSMA, Her2/Neu, EGFRI-III, MUC-1, CD4, cMET, CEA, CD6, CD19, CD22, CD23, CD33, CD44v6, CD56, VEGFRI, VEGFRII, protéine NTS-1, protéine de revêtement gp 120, peptide de fusion gp41, TENB2, HER2/neu (erb2), EGF récepteur vIII, Cripto-1 et Alpha(v)béta(3/5).

10. Oligonucléotide thérapeutique bi-fonctionnel pour utilisation dans le traitement ou la prévention de la maladie selon l'une quelconque des revendications 1 à 9, où l'aptamère de délivrance se lie au PSMA.

11. Oligonucléotide thérapeutique bi-fonctionnel pour utilisation dans le traitement ou la prévention de la maladie selon l'une quelconque des revendications 1 à 10, où l'aptamère de délivrance est A9 (SEQ ID No. 1) ou A10 (SEQ ID No. 2).

12. Oligonucléotide thérapeutique bi-fonctionnel pour utilisation dans le traitement ou la prévention de la maladie selon l'une quelconque des revendications précédentes, où l'ARNsi thérapeutique est spécifique d'une cible sélectionnée dans le groupe consistant en: antigène nucléaire de cellules proliférantes (PCNA), Récepteur Androgène, BCL2, NFkB, VEGFRI, VEGFR2, VEGFR3, HER2/neu, c-Myc, REL-A, PTP1B, BACE, CHK1, PKC-alpha, EGFR, CHK-1, ERG2, Cyclin DI, CCND1 P, KCa, RAF1, MAPK1, MAPK8, MYB, KRAS2, KDR, IKKg, hTR, HRAS, FOS, GAB2, FLT1, EZH2 et CDK2.

13. Oligonucléotide thérapeutique bi-fonctionnel pour utilisation dans le traitement ou la prévention de la maladie selon l'une quelconque des revendications précédentes, où l'ARNsi thérapeutique est spécifique de BCL2.

14. Oligonucléotide thérapeutique bi-fonctionnel pour utilisation dans le traitement ou la prévention de la maladie selon l'une quelconque des revendications précédentes, où la maladie est le cancer.

15. Oligonucléotide thérapeutique bi-fonctionnel comprenant un aptamère de délivrance lié de manière covalente à un ARNsi thérapeutique, où l'aptamère de délivrance se lie à une cible qui est une protéine de surface cellulaire ou un antigène de tumeur.

16. Oligonucléotide thérapeutique bi-fonctionnel selon la revendication 15, qui est constitué d'un aptamère de délivrance et d'un ARNsi thérapeutique.

17. Oligonucléotide thérapeutique bi-fonctionnel selon l'une quelconque des revendications 15 ou 16, où l'ARNsi est lié de manière covalente à l'aptamère de délivrance par une liaison de 5'-3' phosphodiester.

18. Oligonucléotide thérapeutique bi-fonctionnel selon l'une quelconque des revendications 15 à 17, où le brin de codage de l'ARNsi est lié de manière covalente à l'aptamère de délivrance par une liaison de 5'-3' phosphodiester à l'extrémité 5'-3' de l'aptamère de délivrance.

19. Oligonucléotide thérapeutique bi-fonctionnel selon l'une quelconque des revendications 15 à 18, où les brins de codage et de non-codage de l'ARNsi comprennent une séquence contiguë liée de manière covalente à l'aptamère de délivrance par une liaison de 5'-3' phosphodiester à l'un quelconque de l'extrémité 5' ou 3' de l'aptamère de délivrance.

20. Oligonucléotide thérapeutique bi-fonctionnel selon l'une quelconque des revendications 15 à 19, où l'ARNsi est lié de manière covalente à l'aptamère de délivrance par un lieur d'oligonucléotide d'une longueur de dix nucléotides ou moins.

21. Oligonucléotide thérapeutique bi-fonctionnel selon l'une quelconque des revendications 15 à 20, où le fragment de liaison est un fragment d'acide nucléaire, un fragment d'ANP, un fragment peptidique, une liaison disulfure ou un fragment de polyéthylène glycol.

22. Oligonucléotide thérapeutique bi-fonctionnel selon l'une quelconque des revendications 15 à 21 pour l'utilisation dans le traitement du cancer.

23. Oligonucléotide thérapeutique bi-fonctionnel selon la revendication 22, où le traitement implique la modulation de la délivrance intracellulaire de l'ARNsi thérapeutique chez le patient en liant l'aptamère à sa cible pour réguler la délivrance intracellulaire de l'ARNsi thérapeutique.

24. Oligonucléotide thérapeutique bi-fonctionnel selon l'une quelconque des revendications 15 à 23, où l'aptamère de délivrance se lie à une cible sélectionnée dans le groupe consistant en: PSMA, Her2/Neu, EGFRI-III, MUC-1, CD4, cMET, CEA, CD6, CD19, CD22, CD23, CD33, CD44v6, CD56, VEGFRI, VEGFRII, protéine NTS-1, protéine de revêtement gp 120, peptide de fusion gp41, TENB2, HER2/neu (erb2), EGF récepteur vIII, Cripto-1 et Alpha(v)béta(3/5).

25. Oligonucléotide thérapeutique bi-fonctionnel selon l'une quelconque des revendications 15 à 23, où l'aptamère de délivrance se lie au PSMA.

26. Oligonucléotide thérapeutique bi-fonctionnel selon l'une quelconque des revendications 15 à 25, où l'aptamère de délivrance est A9 (SEQ ID No. 1) ou A10 (SEQ ID No. 2).

27. Oligonucléotide thérapeutique bi-fonctionnel selon l'une quelconque des revendications 15 à 26, dans lequel l'oligonucléotide de ARNsi thérapeutique est spécifique d'une cible sélectionnée parmi un antigène nucléaire de cellule proliférante (PCNA), Récepteur d'Androgène, BCL2, NFkB, VEGFRI, VEGFR2, VEGFR3, HER2/neu, c-Myc, REL-A, PTP1B, BACE, CHK1, PKC-alpha, EGFR, CHK-1, ERG2, Cyclin DI, CCND1 P, KCa, RAF1, MAPK1, MAPK8, MYB, KRAS2, KDR, IKKg, hTR, HRAS, FOS, GAB2, FLT1, EZH2 et CDK2.

28. Oligonucléotide thérapeutique bi-fonctionnel selon l'une quelconque des revendications 15 à 27, où la séquence d'oligonucléotides d'ARNsi thérapeutique est spécifique de BCL2.
